(19) Europäisches Patentamt
European Patent Office
Office européen des brevets

(11) EP 2 254 590 B1

(12) **EUROPEAN PATENT SPECIFICATION**

(45) Date of publication and mention of the grant of the patent:
**10.10.2012 Bulletin 2012/41**

(21) Application number: **09700655.5**

(22) Date of filing: **08.01.2009**

(51) Int Cl.:
*A61K 9/16* (2006.01) *A61K 9/50* (2006.01)
*A61K 9/54* (2006.01)

(86) International application number:
**PCT/IL2009/000037**

(87) International publication number:
**WO 2009/087634 (16.07.2009 Gazette 2009/29)**

(54) **METHODS AND COMPOSITIONS FOR ORAL ADMINISTRATION OF INSULIN**

VERFAHREN UND ZUSAMMENSETZUNGEN ZUR ORALEN VERABREICHUNG VON INSULIN

MÉTHODES ET COMPOSITIONS POUR L'ADMINISTRATION ORALE D'INSULINE

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **08.01.2008 IL 18864708**
**14.07.2008 US 80295 P**
**02.10.2008 US 102020**

(43) Date of publication of application:
**01.12.2010 Bulletin 2010/48**

(73) Proprietor: **Oshadi Drug Administration Ltd.**
**76452 Rehovot (IL)**

(72) Inventors:
- **VOL, Alexander**
  **76251 Rehovot (IL)**
- **GRIBOVA, Orna**
  **76452 Rehovot (IL)**

(74) Representative: **Becker Kurig Straus**
**Bavariastrasse 7**
**80336 München (DE)**

(56) References cited:
**US-A- 3 252 859**
**US-A1- 2003 035 888**
**US-A1- 2004 091 541**
**US-A1- 2005 170 004**
**US-A1- 2006 204 559**
**US-A1- 2007 104 778**
**US-A1- 2007 134 332**
**US-A1- 2007 196 656**
**US-A1- 2007 258 889**

- **LIN ET AL.: 'Preparation and Characterization of Nanoparticles Shelled with Chitosan for Oral Insulin Delivery.' BIOMACROMOLECULES vol. 8, no. 1, December 2006, pages 146 - 152, XP008140899**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF INVENTION

The present invention relates to pharmaceutical compositions for oral delivery of insulin, comprising an intimate mixture of solid dry particulate ingredients within an oil. Specifically the pharmaceutical compositions comprise a particulate non-covalently associated intimate mixture of pharmacologically inert silica nanoparticles having a hydrophobic surface, a polysaccharide, and insulin suspended or embedded in an oil or mixture of oils. The present invention further provides methods of manufacturing same and therapeutic uses for oral delivery of insulin.

### BACKGROUND OF THE INVENTION

Oral delivery of active agents is a particularly desirable route of administration, because of safety and convenience considerations and because oral delivery replicates the physiologic mode of insulin delivery. In addition, oral delivery provides for more accurate dosing than multidose vials and can minimize or eliminate the discomfort that often attends repeated hypodermic injections.

There are many obstacles to successful oral delivery of biological macromolecules. For example, biological macromolecules are large and are amphipathic in nature. More importantly, the active conformation of many biological macromolecules may be sensitive to a variety of environmental factors, such as temperature, oxidizing agents, pH, freezing, shaking and shear stress. In planning oral delivery systems comprising biological macromolecules as an active agent for drug development, these complex structural and stability factors must be considered.

In addition, in general, for medical and therapeutic applications, where a biological macromolecule is being administered to a patient and is expected to perform its natural biological function, delivery vehicles must be able to release active molecules, at a rate that is consistent with the needs of the particular patient or the disease process.

The hormone insulin, contributes to the normal regulation of blood glucose levels through its release by the pancreas, more specifically by the B-cells of a major type of pancreatic tissue (the islets of Langerhans). Insulin secretion is a regulated process, which, in normal subjects, provides stable concentrations of glucose in blood during both fasting and feeding. Diabetes is a disease state in which the pancreas does not release insulin at levels capable of controlling glucose levels. Diabetes is classified into two types. The first type is diabetes that is insulin dependent and usually appears in young people. The islet cells of the pancreas stop producing insulin mainly due to autoimmune destruction and the patient must inject himself with the missing hormone. These Type I diabetic patients are the minority of total diabetic patients (up to 10% of the entire diabetic population). The second type of diabetes (type 2) is non-insulin dependent diabetes, which is caused by a combination of insulin resistance and insufficient insulin secretion. This is the most common type of diabetes in the Western world. Close to 8% of the adult population of various countries around the world, including the United States, have Type 2 diabetes, and about 30% of these patients will need to use insulin at some point during their life span due to secondary pancreas exhaustion.

The problem of providing bioavailable unmodified human insulin, in a useful form, to the ever-increasing population of diabetics has occupied physicians and scientists for almost 100 years. Many attempts have been made to solve some of the problems of stability and biological delivery of this small protein. Examples include: US patent 7,455,830 which discloses bioactive nanoparticles suitable for oral delivery of insulin which include a shell substrate of chitosan, and a core substrate selected from the group consisting of gamma-polyglutamic acid (PGA), alpha-PGA, water soluble salts of PGA and metal salts of PGA; US patent 7,470,663 which discloses a liquid solution formulated for oral delivery, comprising a substantially monodispersed mixture of conjugates, wherein each conjugate comprises human insulin covalently coupled a carboxylic acid, which is covalently coupled at the end distal to the carboxylic acid moiety to a methyl terminated polyethylene glycol moiety. US patent 7,384,914 which discloses a method of treating a mammal which has impaired glucose tolerance by administering a therapeutically effective dose of a pharmaceutical formulation comprising insulin and the delivery agent 4-[(2-hydroxy-4-chlorobenzoyl)amino]butanoate (4-CNAB) in an amount which facilitates absorption of the insulin from the gastrointestinal tract of the treated mammal, and US 6,656,922 which discloses a method for enhancing oral administration of insulin by conjugating insulin to an hydrophobic agent selected from the group consisting of bile acids, sterols, alkanoic acids, and mixtures thereof to result in a hydrophobized macromolecular agent.

As of today, most diabetic patients self-administer insulin by daily subcutaneous injections. However, the limitations of multiple daily injections, such as inconvenience, poor patient acceptability, compliance and the difficulty of matching postprandial insulin availability to postprandial requirements are some of the better known shortcomings of insulin therapy.

A method of providing insulin without the need for injections has been a goal in drug delivery. Insulin absorption in the gastrointestinal tract is prevented by its large size and enzymatic degradation. It would be desirable to create an oral pharmaceutical formulation of a drug such as insulin (which is not normally orally administrable due to, e.g., insufficient

absorption from the gastrointestinal tract), which formulation would provide sufficient absorption and pharmacokinetic/pharmacodynamic properties to provide the desired therapeutic effect.

Accordingly, there is a need for a method of administering insulin to patients in need of insulin wherein those patients are not subject to systemic hyperinsulinemia, which by itself can increase the risk of vascular disease (that is normally associated with such chronic insulin treatments, as discussed above). In other words, it is desirable to provide compositions and methods for treating diabetes without the drawbacks of systemic hyperglycemia to decrease the incidence of vascular complications and other detrimental effects.

**Biopolymers and their use in delivering active proteins such as insulin:**

Biopolymers such as polysaccharides have been known for many years. Polysaccharides are widely used as excipients in oral dosage forms, as disclosed for example in US patent 7,351,741 to Weidner, US patent 6,667,060 to Vandecruys and US patent application 2004/0115264 to Blouquin. These references neither disclose nor suggest use of biopolymers in combination with nanoparticles and/or oil.

**Nanoparticles and their use in delivering active proteins such as insulin:**

Silica nanoparticles are well known in the art as pharmaceutical excipients and are their use is disclosed for example in US patents 6,322,765 to Muhlhofer and 6,698,247 to Tennent, among many others. Coating of a nanoparticle-biopolymer complex with oil, or utility of same in oral administration of insulin are neither disclosed nor suggested.

Methods for imparting a hydrophobic surface to nanoparticles are well known in the art and are described, for example, in Chung et al. (Hydrophobic modification of silica nanoparticle by using aerosol spray reactor. Colloids and Surfaces A: Physicochem. Eng. Aspects 236 (2004) 73-79). Additional methods include the reverse micelles method (Fu X, Qutubuddin S, Colloids Surf. A: Physicochem. Eng. Aspects 179: 65, 2001), liquid precipitation method (Krysztatkiewicz A, Jesionowski T, Binkowski S, Colloids Surf. A: Physicochem. Eng. Aspects 173:73, 2000) and sol-gel method (Jean J, Yang S, J. Am. Ceram. Soc. 83(8):1928, 2000; Zhang J, Gao L, Ceram. Int. 27: 143, 2001). Use of nanoparticles in combination with a biopolymer and insulin, coating a nanoparticle-containing complex with oil, or utility of same in oral administration of insulin are neither disclosed nor suggested.

US Patents 7,105,229, 6,989,195, 6,482,517, 6,638,621, 6,458,387, 7,045,146, and 5,462,866 among many others disclose use of nanoparticles or microparticles as excipients for proteins. These references neither disclose nor suggest intimate non-covalent association of nanoparticles with a biopolymer and insulin, or embedding of a nanoparticle-biopolymer-insulin in an oil coating.

US 2007/0154559 to Pai discloses an orally administrable composition containing nanoparticles comprising a charged water-soluble drug in complex with a counter-ion substance, a lipid, a polymer, and an emulsifier. The compositions are formed by (a) ionically bonding the drug with the counter-ion; (b) adding a lipid, a polymer, and a solubilizing agent; dissolving the whole mixture; and introducing the solution into an aqueous solution containing an emulsifier; and (c) removing the solubilizing agent. US 2006/0177495 and 2003/0235619 to Allen disclose delivery vehicles for delivering an active agent, comprising nanoparticles composed of a biodegradable hydrophobic polymer forming a core and an outer amphiphilic layer surrounding the polymer core and containing a stabilizing lipid.

US 2006/0083781 to Shastri discloses nanoparticles comprising a lipid and a polymer comprising an ionic or ionizable moiety. These compositions as well differ significantly from those of the present invention, *inter alia* in that (a) the polymer is not outside the nanoparticles but rather forms a part of them; and (b) the oil forms a part of the nanoparticles instead of coating the nanoparticle-polymer mixture. In addition, the unique structures of the matrix carrier compositions of the present invention is neither disclosed nor suggested.

WO 96/37232 to Alonso Fernandez discloses methods for preparation of colloidal systems through the formation of ionic lipid-polysaccharide complexes. The colloidal systems are stabilized through the formation of an ionic complex, at the interface, comprised of a positively charged aminopolysaccharide and a negatively charged phospholipid. These compositions as well differ significantly from those of the present invention, *inter alia* in that (a) the polymer is not outside the nanoparticles but rather forms a part of them; and (b) the oil forms a part of the nanoparticles instead of coating them. In addition, use of insulin as an active agent is neither disclosed or suggested.

US 6,548,264 to Tan et al. discloses silica-coated nanoparticles and a process for producing silica-coated nanoparticles. Silica-coated nanoparticles are prepared by precipitating nano-sized cores from reagents dissolved in the aqueous compartment of a water-in-oil microemulsion. A reactive silicate is added to coat the cores with silica. The silicate coating may further be derivatized with a protein. US 2007/0275969 to Gurny discloses pharmaceutical compositions for the oral administration of pharmaceutical agents having low water solubility. The pharmaceutical agents are solubilized with a polymer, from which nanoparticles are formed.

In cosmetics formulations, it is common to use compositions comprising water-in-oil emulsions containing an aqueous phase dispersed in an oily phase. There are numerous examples in which silica nanoparticles as well as

polysaccharides are included in the liquid fatty phase. US 6,228,377 for example, discloses water-in-oil emulsions containing a liquid fatty phase which contains hydrophobic or hydrophilic fumed silica, a branched polysaccharide alkyl ether, an emulsifying surfactant and oil. These compositions differ significantly from those of the present invention in that they include a water phase and surfactants that serve as the most important structure forming factor of the composition.

**Additional strategies**

Methods for oral administration of insulin are the object of extensive research efforts but have been proven generally inefficient to date. A number of strategies for preventing degradation of orally administered proteins have been suggested, including use of core-shell particles (US 7,090,868 to Gower) and nano-tubes (US 7,195,780 to Dennis). Liposomes have been used as a carrier for orally administered proteins, as well as aqueous emulsions and suspensions (US 7,316,818; WO 06/062544; US 6,071,535; and US 5,874,105 to Watkins) and gas-filled liposomes (US 6,551,576; US 6,808,720; and US 7,083,572 to Unger et al.). Another composition comprises nanodroplets dispersed in an aqueous medium (US 2007/0184076). Additional strategies are found in WO 06/097793, WO 05/094785, and WO 03/066859 to Ben-Sason, which describe matrix-carriers containing peptide-effectors that provide penetration across biological barriers for administration of hydrophobic proteins; and EP 0491114B1 to Guerrero Gomez-Pamo, which describes preparation of non-covalent protein-polysaccharide complexes for oral administration of biologically active substances, stabilized by precipitates of organic salts. None of these references discloses or suggests intimate non-covalent association of nanoparticles with a biopolymer or a nanoparticles-polymer matrix embedded in an oil coating.

In addition to the differences outlined above, none of the above references discloses or suggests the enhanced bioavailability of compositions of the present invention.

**SUMMARY OF THE INVENTION**

The present invention relates to pharmaceutical compositions for oral delivery of insulin, comprising an intimate mixture of solid dry particulate ingredients within an oil. Preferably the compositions are anhydrous. Specifically the pharmaceutical compositions comprise a particulate non-covalently associated mixture of pharmacologically inert silica nanoparticles having a hydrophobic surface, a polysaccharide, and insulin suspended in, embedded in or dispersed in an oil or mixture of oils. The present invention further provides methods of manufacturing same and therapeutic uses for oral delivery of insulin.

According to the present invention it is now disclosed for the first time that the compositions of the invention surprisingly enable oral bioavailability of insulin. The present invention is based in part on the surprising discovery that experimental diabetic mice treated orally with a composition of the present invention, maintained normal blood glucose levels (~100 mg/dL) for up to 12 hours after administration of the drug whereas diabetic mice given the same amount of insulin by intravenous injection could not maintain a normal blood glucose level for over 6 hours.

In one aspect, the present invention provides a pharmaceutical composition for oral delivery of insulin comprising an oil having particulate matter suspended therein, wherein the particulate matter includes: (a) pharmacologically inert silica nanoparticles having a hydrophobic surface, wherein the size of the nanoparticles is between 1-100 nanometers, in intimate non-covalent association with a polysaccharide; and (b) an insulin protein attached to the silica nanoparticles and the polysaccharide via non-covalent forces. In another embodiment, the insulin protein is attached to the hydrophobic surfaces of the silica nanoparticles and the polysaccharide via non-covalent forces (Figure 1). In another embodiment, the hydrophobic portion of the insulin protein is attached to the hydrophobic surfaces of the silica nanoparticles and the polysaccharide via non-covalent forces. In another embodiment, the hydrophilic portion of the insulin protein is also non-covalently attached to hydrophilic portion of the polysaccharide. In another embodiment, the non-covalent forces cause the nanoparticles, polysaccharide, and insulin to form an intimate mixture. Each possibility represents a separate embodiment of the present invention.

In one preferred embodiment of the present invention, the polysaccharide comprises a branched polysaccharide. In another embodiment, the branched polysaccharide is selected from the group consisting of amylopectin, starch and glycogen. In another embodiment, the branched polysaccharide is starch.

In another embodiment, the particulate matter including the hydrophobic silica nanoparticles, the polysaccharide and the insulin is dispersed in, embedded in or suspended within the oil phase of the matrix composition. In another embodiment, the oil phase is impregnated with the particulate matter. As provided herein, the present invention provides compositions wherein the particulate matter form a matrix that is impregnated and completely surrounded by the oil phase. Preferably the weight of polysaccharides will be greater than the weight of the silica. In some embodiments the weight of the polysaccharides will be at least twice that of the silica, in other embodiments the weight of the polysaccharides will be 5 fold that of the silica in yet other embodiments the polysaccharides will be at least 10 times greater than the weight of silica nanoparticles. Each possibility represents a separate embodiment of the present invention.

Reference to silica nanoparticles of the present invention as having a "hydrophobic" surface encompasses silica nanoparticles having a surface modified to be hydrophobic. In another embodiment, the silica nanoparticles are modified by chemically coating the surface with a hydrocarbon. In another embodiment, the coating causes the silica nanoparticles to display hydrocarbon moieties on their surface. Methods for imparting a hydrophobic surface to silica nanoparticles are well known in the art, and are described *inter alia* herein. Each possibility represents a separate embodiment of the present invention.

In another embodiment, a pharmaceutical composition of the present invention comprises a mixture of oils selected from natural vegetable oils and synthetic analogues thereof.

In another embodiment, a pharmaceutical composition of the present invention further comprises an additional oil component. The term "additional oil component" encompasses an additional oil or mixture of oils, as described elsewhere herein. In another embodiment, the additional oil component comprises an antioxidant. Each possibility represents a separate embodiment of the present invention.

In another embodiment, a pharmaceutical composition of the present invention further comprises a third oil or mixture of oils in addition to the above-described additional oil or mixture of oils. In another embodiment, the third oil component comprises an antioxidant. Each possibility represents a separate embodiment of the present invention.

In another embodiment, a matrix composition of the present invention further comprises a wax.

In another embodiment, a matrix composition formulated for oral administration of the present invention is in the form of a tablet, capsule, or suspension.

In another embodiment, a pharmaceutical composition of the present invention further comprises an additional biopolymer that is a linear biopolymer. In another embodiment, the additional biopolymer is a polysaccharide. In another embodiment, the additional biopolymer is a linear polysaccharide. In another embodiment, the additional biopolymer is a linear high molecular weight structural protein. In another embodiment, the additional biopolymer is selected from the group consisting of chitin, cellulose, a linear alpha glucan, and a linear beta glucan. In another embodiment, the additional biopolymer is selected from the group consisting of chitin, amylose, cellulose, and beta glucan. In another embodiment, a pharmaceutical composition of the present invention comprises a branched polysaccharide and a linear polysaccharide. Each possibility represents a separate embodiment of the present invention.

In another embodiment, the additional biopolymer of uses and compositions of the present invention is a fiber. In another embodiment, the fiber is a dietary fiber. In another embodiment, the dietary fiber is an insoluble fiber. In another embodiment, the dietary fiber is a linear insoluble fiber. In another embodiment, the dietary fiber is a soluble fiber. In another embodiment, the dietary fiber is a linear soluble fiber. Each possibility represents a separate embodiment of the present invention.

In another embodiment, a pharmaceutical composition of the present invention comprises a branched biopolymer, a linear polysaccharide, and an insoluble fiber. In another embodiment, a composition of the present invention comprises a branched polysaccharide, a linear polysaccharide, and an insoluble fiber. An example of such is a composition comprising amylopectin, a branched polysaccharide; chitin, a linear polysaccharide; and cellulose, an insoluble fiber. Other branched and linear polysaccharides and insoluble fibers disclosed herein are suitable as well. Each possibility represents a separate embodiment of the present invention.

In another embodiment, the present invention provides the use of the pharmaceutical composition for orally administering insulin to a subject.

In another embodiment, the present invention provides the use of the pharmaceutical composition in the preparation of a medicament for treating diabetes in a subject, wherein said medicament may be orally administered.. In another embodiment, the diabetes is an insulin-dependent diabetes. In another embodiment, the diabetes is a non-insulin-dependent diabetes. In another embodiment, the diabetes is Type I diabetes. In another embodiment, the diabetes is Type II diabetes. In another embodiment, the diabetes is juvenile diabetes. In another embodiment, the diabetes is adolescent diabetes. In another embodiment, the diabetes is adult diabetes. In another embodiment, the diabetes is any other type of diabetes known in the art. In another embodiment, a complication of diabetes may be treated. Each possibility represents a separate embodiment of the present invention.

The subject of the use of the present invention may be a human. In another embodiment, the subject is a non-human mammal. Each possibility represents a separate embodiment of the present invention.

As provided herein, oral administration of compositions of the present invention lowers blood glucose levels for several hours in animal (Example 6) and human (Example 7) subjects, without causing glycemic instability or troublesome hypoglycemia symptoms. Further, the compositions exhibit no detectable toxicity (Example 8).

In another embodiment, the present invention provides use of a pharmaceutical composition of the present invention in the preparation of a medicament for administering insulin to a subject.

In another embodiment, the present invention provides use of a pharmaceutical composition of the present invention in the preparation of a medicament for treating diabetes in a subject.

In another embodiment, the present invention provides a pharmaceutical composition of the present invention for administering insulin to a subject.

In another embodiment, the present invention provides a pharmaceutical composition of the present invention for treating diabetes in a subject.

In certain embodiments, the insulin in a pharmaceutical composition of the present invention is capable of reaching the bloodstream of a subject, following oral administration, with over 20% of the biological activity intact, preferably over 30% of the biological activity remains intact, more preferably at least 40% of the biological activity remains intact, most preferably at least 50% of the biological activity remains intact. In another embodiment, over 60% of the biological activity remains intact. In another embodiment, over 70% of the biological activity remains intact. In another embodiment, over 80% of the biological activity remains intact. In another embodiment, over 90% of the biological activity remains intact. Without wishing to be bound by any theory or mechanism of action, these properties are believed to be due to protection of the active agent from digestive enzymes and mechanical forces in the intestines by the excipients of pharmaceutical compositions of the present invention.

In another embodiment, a pharmaceutical composition of the present invention is designed to provide short-term release. "Short-term release", as used herein, refers to release over 8-12 hours, with maximal activity 4 hours after administration. In another embodiment, a pharmaceutical composition of the present invention is designed to provide medium-term release. "Medium-term release", as used herein, refers to release over 12-18 hours, with maximal activity 4-6 hours after administration. In another embodiment, a pharmaceutical composition of the present invention is designed to provide long-term release. "Long-term release", as used herein, refers to release over 18-48 hours, with maximal activity 4-8 hours after administration. In another embodiment, a pharmaceutical composition of the present invention is designed to provide very long-term release. "Very long-term release", as used herein, refers to release over 18-72 hours, with maximal activity 6-8 hours after administration. In another embodiment, the longer term-release compositions of the present invention exhibit a lower peak with a longer tail following the peak activity. Each possibility represents a separate embodiment of the present invention.

In another aspect, the present invention provides a method of manufacturing a pharmaceutical composition for oral delivery of insulin, the method comprising the steps of: (a) blending pharmacologically inert silica nanoparticles having a hydrophobic surface, wherein the size of the nanoparticles is between 1-100 nanometers, with a polysaccharide, whereby the silica nanoparticles form an intimate non-covalent association with the polysaccharide; (b) mixing an insulin protein with an oil; and (c) mixing the nanoparticles and polysaccharide into the oil. In another embodiment, the silica nanoparticles, polysaccharide, and insulin thereby form a matrix that becomes dispersed, embedded or suspended in the oil. Preferably, the silica nanoparticles, polysaccharide, and insulin form a complex. In another embodiment, the complex is dispersed, embedded or suspended in the oil. In another embodiment, the insulin protein is non-covalently attached to the hydrophobic surfaces of the silica nanoparticles and to the hydrophilic and hydrophobic portions, regions or patches of the surface of the polysaccharide. In another embodiment, the particle size of the pharmaceutical composition is between 100-500,000 nanometers (nm). In some preferred embodiments, the particle size is between 100-50,000 nm. Each possibility represents a separate embodiment of the present invention.

In yet another aspect, the present invention provides a method of manufacturing a pharmaceutical composition for oral delivery of insulin, the method comprising the steps of: (a) blending pharmacologically inert silica nanoparticles having a hydrophobic surface, wherein the size of the nanoparticles is between 1-100 nanometers, with (i) a polysaccharide, and (ii) an insulin protein whereby the silica nanoparticles form an intimate non-covalent association with the polysaccharide; and (b) mixing the particulate matter (silica nanoparticles, polysaccharide, and insulin protein) into an oil. In another embodiment, the silica nanoparticles, polysaccharide, and insulin form a matrix that becomes dispersed, embedded or suspended in the oil. Preferably, the silica nanoparticles, polysaccharide, and insulin form a complex. In another embodiment, the complex is dispersed, embedded or suspended in the oil. In another embodiment, the insulin protein is non-covalently attached to the hydrophobic surfaces of the silica nanoparticles and to the hydrophilic and hydrophobic portions, regions or patches of the surface of the polysaccharide. In another embodiment, the particle size of the pharmaceutical composition is between 100-500,000 nanometers. In some preferred embodiments, the particle size is between 100-50,000 nanometers. Each possibility represents a separate embodiment of the present invention. As provided herein, methods have been developed to formulate insulin in orally administrable form. In certain preferred embodiments, the components are mixed in a particular order in order to produce oil-coated matrix carrier compositions that protect the insulin from digestive processes in the stomach and small intestine. Further, without wishing to be bound by any theory or mechanism of action, the polysaccharide, particularly when branched, absorbs hydraulic and mechanical stresses experienced during digestion. The oil coating constitutes a physical barrier that provides additional protection against digestive enzymes. The pharmaceutical compositions of the present invention are converted in the digestive system to particles smaller in size but similar in structure to the original composition, which are absorbed similarly to chylomicrons and reach the bloodstream without undergoing first-pass metabolism in the liver. In another embodiment, the particles are broken down in gastro-intestinal tract to particles having a characteristic size between 30-1000 nanometers. In certain preferred embodiments, the size of the particles after digestion is between 30-700 nm. While the primary particles are in the nanometer to sub-micrometer range, these may form conglomerates or agglomerates of larger dimensions within the compositions of the present invention. The size of these conglomerates or agglomerates ranges

between 100-500,000 nanometers. In some preferred embodiments, the conglomerate or agglomerate size is between 100-50,000 nanometers. In another embodiment, the conglomerate or agglomerate size is between 100-5,000 nanometers. Each possibility represents a separate embodiment of the present invention.

## BRIEF DESCRIPTION OF THE DRAWINGS

Figure 1: Schematic view of a representative matrix-carrier structure containing insulin, silica nanoparticles and a polysaccharide. Top: Macrostructure containing branched fiber structure of the polysaccharide impregnated with hydrophobic silica nanoparticles. Bottom: microstructure depiction.

Figure 2: Schematic view of the structure formed in the small intestine due to joint action of hydrodynamic and enzymatic processes.

Figure 3: Light microscopy picture of insulin matrix carrier Formulation IV (Example 5).

Figure 4: A. Effect of oral administration of NovoRapid™ insulin oral composition of the present invention (Formulation VI, Example 4) on blood glucose levels (BGL) in diabetic (STZ-treated) mice. Different symbols represent individual mice. The break indicates the time of the insulin composition administration. **B**. Effect of oral administration of Actrapid™ insulin compositions (Formulation V, Example 4) on BGL in diabetic (STZ-treated) mice. Different symbols represent individual mice.

Figure 5: BGL levels in STZ-treated mice orally receiving 25 IU Insulin (by BIOCON) in PBS (gavage). Different symbols represent individual mice.

Figure 6: **A.** Dose response curve towards the insulin matrix carrier compositions (Formulation IV, Example 5) of the present invention on STZ mice (mean blood glucose concentrations are based on the BGL of at least 5 mice). **B-D.** Data from individual STZ mice administered (Formulation IV): 2 **(B),** 5 **(C),** and 10 **(D)** IU of insulin. **E.** Effect of SC-injected insulin on STZ mice. **F.** Effect of 12 IU of insulin composition on normal mice. The breaks in figures B-I indicate the time of administration. Different symbols represent individual mice. **G, H, I:** Comparison of the effect of 10 **(G),** 5 **(H),** and 2 **(I)** IU of insulin on the BGL upon administration of insulin by SC injection and orally using the matrix carrier composition of the present invention. **J-K.** Comparison of the pharmacodynamics of two different matrix carrier compositions for oral delivery of insulin (formulation A versus formulation IV) - 2 IU of insulin **(J)** and 7.5 IU of insulin **(K).**

Figure 7: Efficacy of oral insulin compositions of the present invention on healthy **(A)** and diabetic **(B)** human subjects. **A.** 30 IU of the Actrapid™ relatively short-term release insulin matrix carrier composition (Formulation II) was administered at time 12:00, as indicated by the stripe in the graph. **B.** Daily average blood glucose levels. Gluco-Rite™ was administered on days 2-12. Insulin matrix carrier composition (formulation V, example 4) was first administered on the 13$^{th}$ day and continued for 14 days.

Figure 8: Toxicity study of insulin oral compositions (Formulation IV) of the present invention. Microscopic analysis of the liver **(A);** kidney **(B);** and duodenum **(C).** In each case, left panels are control samples and right panels are treated samples.

Figure 9: **A-D** Cryo SEM freeze fracture pictures of mice serum taken 4 hours after oral administration (gavage) of 10 IU oral insulin composition (Formulation IV).

## DETAILED DESCRIPTION OF THE INVENTION

The present invention provides matrix carrier compositions for oral delivery of insulin, comprising an intimate mixture of solid dry particulate ingredients within an oil. Specifically the pharmaceutical compositions comprise a particulate non-covalently associated mixture of pharmacologically inert silica nanoparticles having a hydrophobic surface, a branched polysaccharide and insulin suspended, embedded or dispersed in an oil or mixture of oils. The present invention further provides methods of manufacturing same and therapeutic uses for oral delivery of insulin.
The oral insulin compositions of the present invention provide an advantageous result over the subcutaneously administered insulin, which is currently the state of the art, beyond the benefit of ease of administration, pain-free

administration, and the potential for improved patient compliance. By administration of the oral insulin compositions of the present invention, the blood levels of insulin which occur upon the first (initial) phase of insulin secretion by the pancreas can be simulated. The first phase of insulin secretion, while of short duration, has an important role in priming the liver to the metabolic events ahead (meal). Because subcutaneously administered insulin does not undergo portal circulation, this result is not possible with subcutaneously administered insulin.

In another embodiment, the present invention provides a pharmaceutical composition comprising: (a) pharmacologically inert silica nanoparticles having a hydrophobic surface, wherein the diameter of the nanoparticles is between 1-100 nanometers, in intimate mixture with a polysaccharide; and (b) an insulin protein non-covalently attached to the silica nanoparticles and the polysaccharide; wherein the matrix formed by the silica nanoparticles, polysaccharide, and insulin is suspended, embedded or dispersed in oil. In another embodiment, the insulin is non-covalently attached to the hydrophobic surfaces of the silica nanoparticles and to the hydrophilic and hydrophobic portions, regions or patches of the surface of the polysaccharide. In another embodiment, the hydrophobic portion of the insulin protein is attached to the hydrophobic surfaces of the silica nanoparticles and the polysaccharide via non-covalent forces. In another embodiment, the hydrophilic portion of the insulin protein is also non-covalently attached to hydrophilic portion of the polysaccharide. In another embodiment, the particle diameter of the pharmaceutical composition following its formation, but prior to ingestion is between 100-500,000 nm. In certain preferred embodiments, the particle diameter is between 100-50,000 nanometers. In another embodiment, the particle diameter is between 100-5000 nm. Each possibility represents a separate embodiment of the present invention.

Various components of pharmaceutical compositions of the present invention, namely insulin, silica nanoparticles, polysaccharides, high molecular weight structural proteins, and oils, are described herein. Each embodiment thereof can be utilized in the therapeutic uses of the present invention, and each such use represents a separate embodiment of the present invention.

In another embodiment, the oil phase of the matrix carrier composition comprises a plurality of oils.

In another embodiment, a pharmaceutical composition of the present invention is held together by non-covalent forces (Figure 1). In another embodiment, without wishing to be bound by any theory or mechanism of action, the non-covalent forces between the components of the matrix composition enable the matrix composition to self-assemble when the components are mixed together, as described herein. In another embodiment, the non-covalent forces cause the silica nanoparticles, polysaccharide and insulin to form an intimate mixture. In another embodiment, the matrix composition exhibits an ordered structure. In another embodiment, without wishing to be bound by any theory or mechanism of action, the matrix composition includes a solid phase containing at least two solid pharmacologically inert materials (silica nanoparticles and polysaccharides) with different properties. In another embodiment, the silica nanoparticle/polysaccharide/insulin complex is dispersed, embedded or suspended within the oil phase of the matrix composition. In another embodiment, the oil phase is impregnated with the silica nanoparticle/polysaccharide/insulin complex of the matrix composition. As provided herein, the present invention provides compositions wherein the silica nanoparticles, polysaccharide, and insulin form a matrix that is impregnated and completely surrounded by the oil phase. Each possibility represents a separate embodiment of the present invention.

In another embodiment, a pharmaceutical composition of the present invention further comprises an additional biopolymer that is a linear biopolymer. In another embodiment, the additional biopolymer is a linear polysaccharide. In another embodiment, the additional biopolymer is a linear high molecular weight structural protein. In another embodiment, the additional biopolymer is selected from the group consisting of chitin, cellulose, a linear alpha glucan, and a linear beta glucan. In another embodiment, the additional biopolymer is selected from the group consisting of chitin, amylose, cellulose, and beta glucan. Insulin compositions are provided herein that comprise amylopectin, a branched biopolymer, and chitin, a linear biopolymer (Example 5). Other branched and linear biopolymers disclosed herein are suitable as well. Each possibility represents a separate embodiment of the present invention.

In another embodiment, the additional biopolymer of uses and compositions of the present invention is a dietary fiber. In another embodiment, the dietary fiber is an insoluble fiber. In another embodiment, the dietary fiber is a linear insoluble fiber. In another embodiment, the dietary fiber is a soluble fiber. In another embodiment, the dietary fiber is a linear soluble fiber.

In another embodiment, a composition of the present invention comprises a branched biopolymer, a linear polysaccharide, and an insoluble fiber. In another embodiment, a composition of the present invention comprises a branched biopolymer, a polypeptide, and an insoluble fiber. An example of such is a composition comprising amylopectin, a branched polysaccharide; keratin, a polypeptide; and cellulose, an insoluble fiber. Other branched polysaccharides, polypeptides, and insoluble fibers disclosed herein are suitable as well. In another embodiment, a composition of the present invention comprises a branched polysaccharide, a linear polysaccharide, and an insoluble fiber. An example of such is a composition comprising amylopectin, a branched polysaccharide; chitin, a linear polysaccharide; and cellulose, an insoluble fiber. Other branched and linear polysaccharides and insoluble fibers disclosed herein are suitable as well. Each possibility represents a separate embodiment of the present invention.

Oil having particulate matter suspended therein, as used herein, refers to particulate matter that is in contact

with oil. The composition as a whole need not be homogeneous with regard to the distribution of the particulate matter. Rather, the particulate matter is capable of being dispersed or suspended in the oil when agitated. The particulate matter need not be completely homogeneous, but rather is characterized by its containing the ingredients specified herein and its intimate contact with the oil of the present invention. Compositions wherein the particulate matter is agglomerated fall within the scope of the present invention.

**Nanoparticles**

The silica nanoparticles of uses and compositions of the present invention are preferably pharmacologically inert. In another embodiment, the silica nanoparticles are composed of materials that are generally recognized as safe (GRAS). In another embodiment, the silica nanoparticles are non-toxic. In another embodiment, the silica nanoparticles are non-teratogenic. In another embodiment, the silica nanoparticles are biologically inert. Each possibility represents a separate embodiment of the present invention.

In another embodiment, the nanoparticles are silica-containing nanoparticles. "Silica-containing nanoparticles" refers preferably to nanoparticles comprising silica, a silicate, or a combination thereof. "Silica" refers to silicon dioxide. Silica-containing nanoparticles are available commercially, e.g. as 99.99% pure finely ground silica. It will be understood by those skilled in the art that lower grades of purity of silica are also compatible with the present invention. "Silicate" refers to a compound containing silicon and oxygen, e.g. in tetrahedral units of $SiO_4$. In another embodiment, the term refers to a compound containing an anion in which one or more central silicon atoms are surrounded by electronegative ligands. Examples of silicates are hexafluorosilicate, sodium silicate ($Na_2SiO_3$), aluminum silicates, magnesium silicates, etc. It is to be understood that the nanoparticles in structures of the present invention can be either of a single type or of multiple types, provided that, if multiple types are present, at least one type is a silica-containing nanoparticles. In another embodiment, essentially all the nanoparticles are silica-containing nanoparticles. Silica is widely recognized as a safe food additive (Thirteenth report of the Joint FAO/WHO Expert Committee on Food Additives, FAO Nutrition Meetings Report Series; from the Joint FAO/WHO Expert Committee on Food Additives meeting in Rome, May 27- June 4, 1969). Each possibility represents a separate embodiment of the present invention.

Reference to silica nanoparticles of the present invention as having a "hydrophobic" surface indicates, in one embodiment, that at least 40% of the silica nanoparticle surface is hydrophobic. In another embodiment, at least 50% of the surface is hydrophobic. In another embodiment, at least 60% of the surface is hydrophobic. In another embodiment, at least 70% of the surface is hydrophobic. In another embodiment, at least 80% of the surface is hydrophobic. In another embodiment, at least 90% of the surface is hydrophobic. In another embodiment, at least 95% of the surface is hydrophobic. In another embodiment, 40-100% of the surface is hydrophobic. In another embodiment, 50-100% of the surface is hydrophobic. In another embodiment, 60-100% of the surface is hydrophobic. In another embodiment, 70-100% of the surface is hydrophobic. In another embodiment, 80-100% of the surface is hydrophobic. In another embodiment, 90-100% of the surface is hydrophobic. In another embodiment, 95-100% of the surface is hydrophobic. In another embodiment, 40-60% of the surface is hydrophobic. In another embodiment, 40-50% of the surface is hydrophobic. In another embodiment, 40-70% of the surface is hydrophobic. In another embodiment, 40-80% of the surface is hydrophobic. Each possibility represents a separate embodiment of the present invention.

In another embodiment, reference to silica nanoparticles as having a "hydrophobic" surface encompasses silica nanoparticles having a surface chemically modified to be hydrophobic. In another embodiment, the nanoparticles are chemically modified by coating the surface with a hydrocarbon. In another embodiment, the coating causes the nanoparticles to display hydrocarbon moieties on their surface. In another embodiment, the hydrocarbon moieties are selected from the group consisting of methyl, ethyl, propyl, isopropyl, butyl, isobutyl, T-butyl, pentyl, and iso-pentyl. In another embodiment, the coating causes the nanoparticles to display methyl moieties on their surface. Methods for imparting a hydrophobic surface to nanoparticles are well known in the art, and are described *inter alia* herein. As is known in the art it is possible to chemically modify the surface of the fumed silica by chemical reaction, generating a decrease in the number of silanol groups. In particular, silanol groups can be substituted with hydrophobic groups to obtain a hydrophobic silica. The hydrophobic groups can be: trimethylsiloxy groups, which are obtained in particular by treatment of fumed silica in the presence of hexamethyldisilazane. Silicas thus treated are known as "silica silylate" according to the CTFA (6th edition, 1995). They are sold, for example, under the references "Aerosil R812®" by the company Degussa and "CAB-OSIL TS-530®" by the company Cabot; dimethylsilyloxy or polydimethylsiloxane groups, which are obtained in particular by treatment of fumed silica in the presence of polydimethylsiloxane or dimethyldichlorosilane. Silicas thus treated are known as "silica dimethyl silylate" according to the CTFA (6th edition, 1995). They are sold, for example, under the references "Aerosil R972®.", "Aerosil R974®" by the company Degussa, "CAB-O-SIL TS-610®." and "CAB-O-SIL TS-720®." by the company Cabot. Each possibility represents a separate embodiment of the present invention.

In another embodiment, nanoparticles of compositions of the present invention are practically insoluble in water. "Practically insoluble" refers, in another embodiment, to a substance having a solubility of less than 100 parts per million weight/weight (ppm). In another embodiment, the term refers to a solubility of less than 200 ppm. In another embodiment,

the term refers to a solubility of less than 80 ppm. In another embodiment, the term refers to a solubility of less than 60 ppm. In another embodiment, the term refers to a solubility of less than 50 ppm. In another embodiment, the term refers to a solubility of less than 40 ppm. In another embodiment, the term refers to a solubility of less than 30 ppm. In another embodiment, the term refers to a solubility of less than 20 ppm. In another embodiment, the term refers to a solubility of less than 15 ppm. In another embodiment, the term refers to a solubility of less than 10 ppm. Each possibility represents a separate embodiment of the present invention.

In another embodiment, the diameter of the silica nanoparticles of uses and compositions of the present invention is between 5-30 nanometers inclusive. In another embodiment, the diameter is between 2-400 nanometers (nm) inclusive. In another embodiment, the diameter is between 2-300 nm inclusive. In another embodiment, the diameter is between 3-200 nm inclusive. In another embodiment, the diameter is between 4-150 nm inclusive. In another embodiment, the diameter is between 4-100 nm inclusive. In another embodiment, the diameter is between 5-50 nm inclusive. In another embodiment, the diameter is between 5-40 nm inclusive. In another embodiment, the diameter is between 6-25 nm inclusive. In another embodiment, the mean diameter of silica nanoparticles is 10-11 nm.

In another embodiment, the average diameter is about 5 nm. In another embodiment, the average diameter is about 6 nm. In another embodiment, the average diameter is about 7 nm. In another embodiment, the average diameter is about 8 nm. In another embodiment, the average diameter is about 9 nm. In another embodiment, the average diameter is about 10 nm. In another embodiment, the average diameter is about 12 nm. In another embodiment, the average diameter is about 14 nm. In another embodiment, the average diameter is about 16 nm. In another embodiment, the average diameter is about 18 nm. In another embodiment, the average diameter is about 20 nm. In another embodiment, the average diameter is another diameter falling within a range disclosed herein. Each possibility represents a separate embodiment of the present invention.

In another embodiment, silica nanoparticles of the present invention fall within a range of melting temperatures particularly suitable for compositions of the present invention. In specific embodiments, the silica nanoparticles have a melting temperature ($T_m$) of over 600 °C. In another embodiment, the $T_m$ is between 600-4500°C. In another embodiment, the $T_m$ is another $T_m$ falling within a range disclosed herein. Each possibility represents a separate embodiment of the present invention.

**Imparting a hydrophobic surface to a nanoparticle**

Methods for imparting a hydrophobic surface to nanoparticles are well known in the art and are described, *inter alia,* in Chung et al (Hydrophobic modification of silica nanoparticle by using aerosol spray reactor. Colloids and Surfaces A: Physicochem. Eng. Aspects 236 (2004) 73-79). Additional methods include the reverse micelles method (Fu X, Qutubuddin S, Colloids Surf. A: Physicochem. Eng. Aspects 179: 65, 2001), liquid precipitation method (Krysztafkiewicz A, Jesionowski T, Binkowski S, Colloids Surf. A: Physicochem. Eng. Aspects 173:73, 2000) and sol-gel method (Jean J, Yang S, J. Am. Ceram. Soc. 83(8):1928, 2000; Zhang J, Gao L, Ceram. Int. 27: 143, 2001). US 2007/0172426 provides additional methods of imparting a hydrophobic surface to a nanoparticle, by combining them with a material having a first end that adsorbs to the surface of the nanoparticle and a second end that extends away from the nanoparticle and imparts hydrophobicity to the particles. The material may be a generally aliphatic compound having a polar end-group. The first end of each molecule of the compound may include a carboxyl group, an amine group, a silane, etc., that adsorbs to the surface of the particle. The second end of each molecule of the compound may include alkane group that extends away from the particle. Materials used to provide the hydrophobic surface layer include saturated fatty acids such as lauric acid, myristic acid, palmitic acid, and stearic acid, and unsaturated variants thereof, such as palmitoleic acid, oleic acid, linoleic acid, and linolenic acid. Silanes such as octadecyl trichlorosilane can also be widely used to functionalize oxide surfaces. The hydrophobic surface layer is provided by mixing the nanoparticles into a volume of hydrophobic coating material suitable for coating the particles. An excess of hydrophobic coating material is generally used so that the nanoparticles form a suspension in the hydrophobic coating material. Each nanoparticle then exhibits a hydrophobic layer on its surface. Additional methods for utilizing a hydrocarbon surfactant to coat nanoparticles are described in US 2006/0053971. Additional methods are described in US 2007/0098990. The disclosed methods utilize multiple organic acids in which the first acid is a low molecular weight organic carboxylic acid and the second acid is a high molecular weight organic carboxylic acid. The contents of each of the above patent applications are hereby incorporated by reference.

**Biopolymers**

Uses and compositions of the present invention preferably comprise a branched biopolymer. "Branched" as used herein encompasses both polymers that are naturally branched and those engineered to be branched by physical treatment such as thermal and/or ultrasound treatment. In general, branched polymers are defined as polymers wherein a monomer subunit is covalently bound to more than two monomer subunits. Such a monomer is the site of a branch

point, wherein multiple polymer chains converge. In another embodiment, the branched biopolymer is a crosslinked polymer. In another embodiment, the branched biopolymer is not crosslinked. Examples of branched polymers are glycogen and amylopectin, forms of starch found in animals and plants, respectively. Structures of amylopectin (CAS# 9037-22-3) and glycogen (CAS# 9005-79-2) are depicted below:

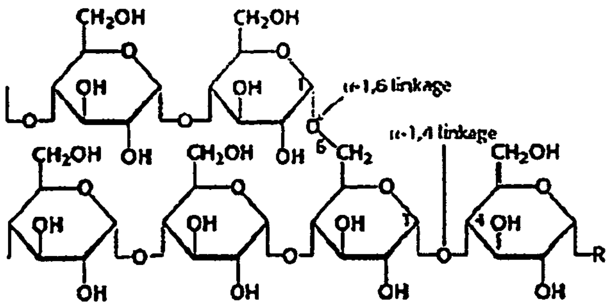

Glycogen

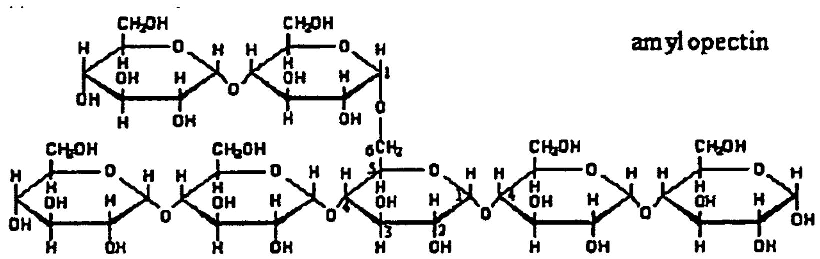

amylopectin

In another embodiment, the biopolymer is a fibrous biopolymer. "Fibrous polymer" refers to a polymer in the form of a network of discrete thread-shaped pieces. Examples of fibrous polymers are guar gum (found for example in Benefiber™), collagen, keratin, fibrin, and elastin. Biopolymers can be either naturally fibrous or made fibrous by physical and chemical treatment.

In another embodiment, the biopolymer is a fiber. "Fiber" refers, in another embodiment, to an indigestible component that acts as a bulking agent for feces. In another embodiment, the fiber is an insoluble fiber. In another embodiment, the fiber is a soluble fiber. Each possibility represents a separate embodiment of the present invention.

Each type of fiber and type of branched and fibrous biopolymer represents a separate embodiment of the present invention.

In another embodiment, the biopolymer is pharmacologically inert. In another embodiment, the biopolymer is non-toxic. In another embodiment, the biopolymer is non-teratogenic. In another embodiment, the biopolymer is biologically inert. Each possibility represents a separate embodiment of the present invention.

In another embodiment, the melting temperature of the biopolymer falls within a range particularly suitable for compositions of the present invention. In another embodiment, the biopolymer has a melting temperature under 400 °C. In another embodiment, the $T_m$ is below 350 °C. In another embodiment, the $T_m$ is below 300 °C. In another embodiment, the $T_m$ is below 250 °C. In another embodiment, the $T_m$ is below 200 °C. In another embodiment, the $T_m$ is below 150 °C. In another embodiment, the $T_m$ is between 100-400 °C. In another embodiment, the $T_m$ is any $T_m$ falling within a range disclosed herein. Each possibility represents a separate embodiment of the present invention.

Preferably, the biopolymer of uses and compositions of the present invention is selected from the group consisting of a polysaccharide and a structural protein.

## Polysaccharides

"Saccharide" refers to any simple carbohydrate including monosaccharides, monosaccharide derivatives, monosaccharide analogs, sugars, including those, which form the individual units in a polysaccharide. "Monosaccharide" refers to polyhydroxyaldehyde (aldose) or polyhdroxyketone (ketose) and derivatives and analogs thereof.

"Polysaccharide" refers to polymers formed from about 500 saccharide units linked to each other by hemiacetal or glycosidic bonds. Typically, polysaccharides can contain as many as 100,000 saccharide units, and in some cases even more. The polysaccharide may be either a straight chain, singly branched, or multiply branched wherein each branch may have additional secondary branches, and the monosaccharides may be standard D- or L-cyclic sugars in

the pyranose (6-membered ring) or furanose (5-membered ring) forms such as D-fructose and D-galactose, respectively, or they may be cyclic sugar derivatives, for example amino sugars such as D-glucosamine, deoxy sugars such as D-fucose or L-rhamnose, sugar phosphates such as D-ribose-5-phosphate, sugar acids such as D-galacturonic acid, or multi-derivatized sugars such as N-acetyl-D-glucosamine, N-acetylneuraminic acid (sialic acid), or N-sulfato-D-glucosamine. When isolated from nature, polysaccharide preparations comprise molecules that are heterogeneous in molecular weight. Polysaccharides include, among other compounds, galactomanans and galactomannan derivatives; galacto-rhamnogalacturons and galacto-rhamnogalacturon derivatives, and galacto-arabinogalacturon and galacto-arabinogalacturon derivatives.

In another embodiment, the polysaccharide of uses and compositions of the present invention is a naturally-occurring polysaccharide. In another embodiment, the polysaccharide is a synthetic polysaccharide. Examples of synthetic polysaccharides can be found in US 6,528,497 and in Okada M. et al. Polymer journal, 15 (11); 821-26 (1983). In another embodiment, the polysaccharide is a naturally-occurring branched polysaccharide. In another embodiment, the polysaccharide is a synthetic branched polysaccharide. Each possibility represents a separate embodiment of the present invention.

In another embodiment, the polysaccharide is a branched polysaccharide. This term is well understood to those skilled in the art and can refer to any number and structure of branches in the links between monosaccharide monomers. In another embodiment, the polysaccharide is a naturally-occurring branched polysaccharide. In another embodiment, the branched polysaccharide is a starch. In another embodiment, the branched polysaccharide is selected from the group consisting of amylopectin, glycogen, and a branched alpha glucan. In another embodiment, the polysaccharide is a synthetic branched polysaccharide. Each possibility represents a separate embodiment of the present invention.

In another embodiment, the polysaccharide is an amphipathic polysaccharide. This term is well understood to those skilled in the art and refers to the existence of both hydrophobic and hydrophilic regions on the polysaccharide. In another embodiment, the polysaccharide is a naturally-occurring amphipathic polysaccharide. Each possibility represents a separate embodiment of the present invention.

In another embodiment, the average MW of the polysaccharide is at least 100 kilodalton (kDa). In another embodiment, the average MW is at least 150 kDa. In another embodiment, the average MW is at least 200 kDa. In another embodiment, the average MW is at least 300 kDa. In another embodiment, the average MW is at least 400 kDa. In another embodiment, the average MW is at least 500 kDa. In another embodiment, the average MW is at least 600 kDa. In another embodiment, the average MW is at least 800 kDa. In another embodiment, the average MW is at least 1000 kDa. In another embodiment, the average MW is between 100-1000 kDa. In another embodiment, the average MW is between 150-1000 kDa. In another embodiment, the average MW is between 200-1000 kDa. In another embodiment, the average MW is between 100-800 kDa. In another embodiment, the average MW is between 100-600 kDa. Each possibility represents a separate embodiment of the present invention.

In another embodiment, the polysaccharide is selected from the group consisting of starch, dextrin, cellulose, chitin, a branched alpha glucan, a branched beta glucan and derivatives thereof. Cellulose, dextrin, starch and glycogen are all polymers of glucose and thus have the formula $(C_6H_{10}O_5)_n$.

In another embodiment, the polysaccharide is a starch, which has the structure below. Examples of starch are corn starch, potato starch, rice starch, wheat starch, purum starch, and starch from algae. In another embodiment, the starch is any other starch known in the art. Each possibility represents a separate embodiment of the present invention.

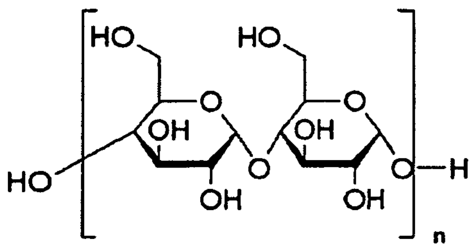

In another embodiment, the polysaccharide is a dextrin. "Dextrin" in another embodiment refers to a low-molecular-weight carbohydrate produced by the hydrolysis of starch. In another embodiment, the term refers to a linear α-(1,4)-linked D-glucose polymer starting with an α-(1,6) bond or a mixture of same. Dextrins are widely commercially available and can be produced *inter alia* by digestion of branched amylopectin or glycogen with α-amylase. An example of a dextrin is a maltodextrin having the structure below. In another embodiment, the dextrin is any other dextrin known in the art. Each possibility represents a separate embodiment of the present invention.

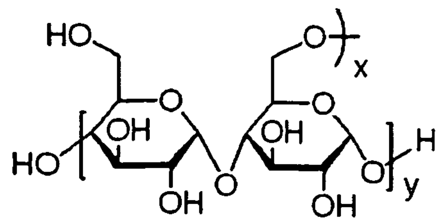

In another embodiment, the polysaccharide is cellulose. An example of a cellulose is α-cellulose, which has the structure below.

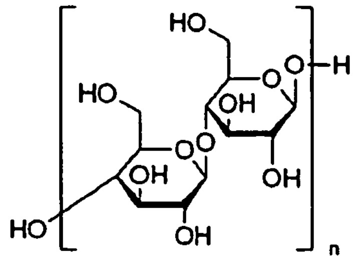

In another embodiment, the polysaccharide is β-cellulose, a linear polymer of D-glucose linked by β(1→4) glycosidic bonds. In another embodiment, the β-cellulose has the structure below.

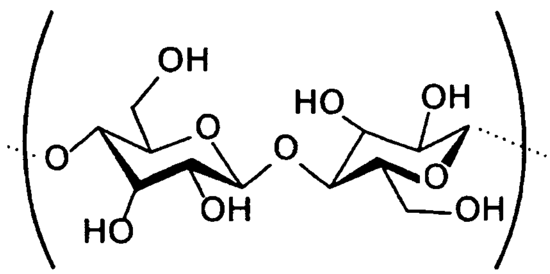

In another embodiment, the cellulose is any other cellulose known in the art. Each possibility represents a separate embodiment of the present invention.

In another embodiment, the polysaccharide is chitin, a long-chain polymer of N-acetylglucosamine, a derivative of glucose. Typically, chitin has the molecular formula $(C_8H_{13}NO_5)_n$ and the structure below. Each possibility represents a separate embodiment of the present invention.

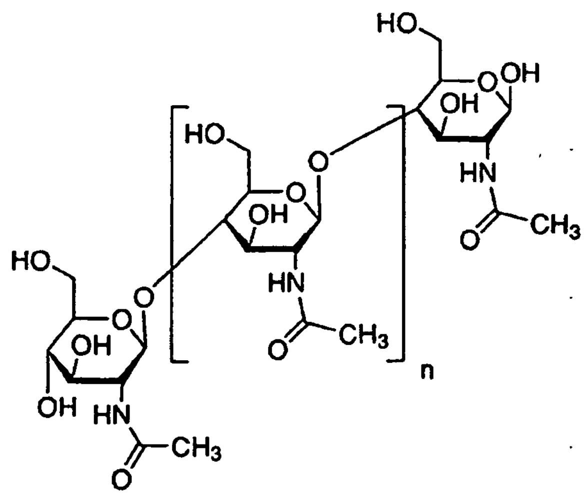

In another embodiment, the polysaccharide is an alpha-glucan. Alpha-glucans of the present invention may be linear or branched polymers of glucose with alpha 1-2, alpha 1-3, alpha 1-4, and/or alpha 1-6 glycosidic linkages. In another embodiment, the alpha-glucan has unbranched linear glucose polymers with 1-4 glycosidic linkages, an example of which is alpha-amylose. In another embodiment, the alpha-glucan has branched glucose polymers with alpha 1-4 glycosidic linkages in the backbone and alpha 1-6 linkages at branch points, an example of which is amylopectin. In another embodiment, the alpha-glucan is any other type of alpha-glucan known in the art. Each possibility represents a separate embodiment of the present invention.

In another embodiment, the polysaccharide is a beta-glucan. "Beta-glucan" refers to polysaccharides containing D-glucopyranosyl units linked together by (1 → 3) or (1 → 4) beta-linkages. Beta-Glucans occur naturally in many cereal grains such as oats and barley. The molecular weight of beta-glucan molecules occurring in cereals is typically 200 to 2000 kDa; other types contain up to about 250,000 glucose units. In another embodiment, the beta-glucan is any other beta-glucan known in the art. Each possibility represents a separate embodiment of the present invention.

In another embodiment, a pharmaceutical composition of the present invention comprises a branched polysaccharide and a linear polysaccharide. In another embodiment, the linear polysaccharide is selected from the group consisting of chitin, cellulose, amylose, and beta glucan. In some preferred embodiments, the branched and linear polysaccharides both have a melting temperature under 400 °C. Insulin compositions are provided herein that comprise amylopectin, a branched polysaccharide, and chitin, a linear polysaccharide (Example 5, Formulation IV). Other branched polysaccharides and linear polysaccharides disclosed herein are suitable as well.

In another embodiment, the additional biopolymer of uses and compositions of the present invention is a fiber, preferentially a dietary fiber. The definition of the term "fiber" and "dietary fiber" as used herein includes unavailable carbohydrates, indigestible residue, and plant cell polysaccharides and lignin, all of which are resistant to hydrolysis by human digestive enzymes. Preferred fibers are members selected from the group consisting of guar gum, pectin, fructo-oligosaccharides and derivatives thereof. Small amounts of other indigestible compounds, such as phytates, tannins, saponins and cutin, may be included in dietary fiber since these compounds are indigestible and associated with dietary fiber polysaccharides. In another embodiment, the dietary fiber is an insoluble fiber. In another embodiment, the dietary fiber is a linear insoluble fiber. In another embodiment, the dietary fiber is a soluble fiber. In another embodiment, the dietary fiber is a linear soluble fiber. Each possibility represents a separate embodiment of the present invention.

In another embodiment, the $T_m$ of a polysaccharide of a composition of the present invention falls within a range of melting temperatures particularly suitable for compositions of the present invention. In another embodiment, the polysaccharide has a $T_m$ under 400 °C. In another embodiment, the $T_m$ is another $T_m$ or range of $T_m$ defined herein. Each possibility represents a separate embodiment of the present invention.

**Structural proteins**

According to certain embodiments the dry solid particulate ingredients of compositions may further comprise a structural protein the structural protein of uses and compositions of the present invention is a high molecular weight (MW) structural protein. In some embodiments, the structural protein comprises both hydrophilic and hydrophobic residues that interact with the hydrophobic and hydrophilic regions, respectively, of the biologically active protein or peptide. In another embodiment, the average MW of the structural protein is at least 100 kilodalton (kDa). In another embodiment, the average MW is at least 150 kDa. In another embodiment, the average MW is at least 200 kDa. In another embodiment, the average MW is at least 300 kDa. In another embodiment, the average MW is at least 400 kDa. In another embodiment, the average MW is at least 500 kDa. In another embodiment, the average MW is at least 600 kDa. In another embodiment, the average MW is at least 800 kDa. In another embodiment, the average MW is at least 1000 kDa. In another embodiment, the average MW is between 100-1000 kDa. In another embodiment, the average MW is between 150-1000 kDa. In another embodiment, the average MW is between 200-1000 kDa. In another embodiment, the average MW is between 100-800 kDa. In another embodiment, the average MW is between 100-600 kDa. Each possibility represents a separate embodiment of the present invention.

"Structural protein", in one embodiment, refers to a protein included for the structure it confers to the matrix carrier composition. In another embodiment, a structural protein of the present invention lacks therapeutic activity. In another embodiment, the term refers to a protein that confers structure to a cell, cellular membrane, or extracellular membrane *in vivo.* In another embodiment, the structural protein is a fibrous protein. In another embodiment, the structural protein is a scleroprotein. In another embodiment, the structural protein is selected from the group consisting of elastin, collagen, keratin, and fibrinogen. In another embodiment, the structural protein is any other fibrous protein or scleroprotein known in the art. Each possibility represents a separate embodiment of the present invention.

In another embodiment, the structural protein is elastin. Examples of elastin proteins are described, *inter alia,* in GenBank Accession numbers NP_031951, NP_786966, and AAC98394. In another embodiment, the elastin is any other elastin known in the art. Each possibility represents a separate embodiment of the present invention.

In another embodiment, the structural protein is collagen. Examples of collagen proteins include those encoded

by gene symbols COL3A1, COL14A1, COLL11A2, COL5A2, COL11A1, COL5A1, COL4A6, COL4A5, COL4A4, COL4A3, COL4A2, COL1A2, COL5A3, COL18A1, COL12A1, COL19A1, COL24A1, COL4A1, and COL2A1. In another embodiment, the collagen is any other collagen known in the art. Each possibility represents a separate embodiment of the present invention.

In another embodiment, the structural protein is keratin. Examples of keratin proteins include keratin 18, keratin 14, keratin 3, and keratin 86 (GenBank Accession numbers P05783, P02533, P12035, 043790, respectively. In another embodiment, the keratin is any other keratin known in the art. Each possibility represents a separate embodiment of the present invention.

In another embodiment, the structural protein is fibrinogen. Fibrinogen is a glycoprotein composed of three pairs of polypeptides: two alpha, two beta, and two gamma chains. Examples of the fibrinogen alpha, beta, and gamma chains are described, *inter alia,* in GenBank Accession numbers P02671, P02675, and P02679. In another embodiment, the fibrinogen is any other fibrinogen known in the art. Each possibility represents a separate embodiment of the present invention.

In another embodiment, the $T_m$ of a structural protein of a composition of the present invention falls within a range of melting temperatures particularly suitable for compositions of the present invention. In another embodiment, the structural protein has a $T_m$ under 400 °C. Each possibility represents a separate embodiment of the present invention.

**Oils and oil coatings**

The particulate matter of matrix compositions of the present invention is surrounded by, suspended in, immersed in, embedded in or dispersed in oil carrier. Typically, the oil phase, in addition to coating the particulate matter, impregnates the particulate matter, which is composed of the silica nanoparticles, branched polysaccharide and insulin. Reference to an "oil," "oil layer," "oil phase," or "oil coating" does not preclude the presence of an additional component or components useful in the therapeutic use of the present invention (e.g. a fat-soluble co-factor or anti-oxidant). Rather, the term indicates that the oil, oil layer, oil phase, or coating is composed primarily of a pharmaceutically acceptable oil carrier, in which the other components are mixed and/or dissolved. The oil carrier can be composed of either one or a plurality of types of oils, as described further herein. In another embodiment, the coating consists essentially of lipids and/or oils. In another embodiment, the coating of the composition comprises a pharmaceutically acceptable oil carrier. In another embodiment, the oil carrier is a naturally occurring oil. In another embodiment, the oil is a mixture of natural vegetable oils. In another embodiment, the oil carrier is sesame oil. In another embodiment, the oil carrier is olive oil. In another embodiment, the oil carrier is linseed oil. In another embodiment, the oil carrier is evening primrose oil. In another embodiment, the oil carrier is silicone oil. In another embodiment, the oil carrier is sea buckthorn oil. In another embodiment, the oil carrier is selected from the group consisting of sesame oil, olive oil, linseed oil, evening primrose oil, silicone oil, and sea buckthorn oil. In another embodiment, the oil carrier includes an oil selected from the group consisting of sunflower oil, corn oil, soybean oil, jojoba oil, marrow oil, grapeseed oil, hazelnut oil, apricot oil, macadamia oil and castor oil. In another embodiment, the oil carrier is another suitable oil known in the art. In another embodiment, the oil carrier is of animal origin, such as lanolin. In another embodiment, the oil carrier is a synthetic oil. In another embodiment, the oil carrier is a fatty alcohol. In certain preferred embodiments, the oil carrier is 2-octyldodecanol. In certain other preferred embodiments, the oil carrier is selected from the group consisting of a fatty acid ester and a phenylsilicone. In certain more preferred embodiments, the oil carrier is selected from the group consisting of a phenyltrimethicone, a diphynyldimethicone, and a poly-methylphenylsiloxane. Each possibility represents a separate embodiment of the present invention.

In another embodiment, the oil consists essentially of naturally-occurring lipids and/or oils. Each possibility represents a separate embodiment of the present invention.

"Plurality of oils" refers, in another embodiment, to two or more oils. In another embodiment, a composition of the present invention comprises three or more oils. In another embodiment, a composition of the present invention comprises four or more oils. In another embodiment, a composition of the present invention comprises more than four oils. In another embodiment, the oil phase comprises a mixture of oils selected from natural vegetable oils. Each possibility represents a separate embodiment of the present invention.

In another embodiment, an oil component of the present invention comprises a component capable of stimulating secretion of bile salts or bile acids when ingested by a subject. In another embodiment, the bile-stimulating component is an oil. In another embodiment, the component is olive oil or an extract thereof. In another embodiment, the component is any other bile salt/acid stimulating lipid-soluble substance known in the art. In another embodiment, the carrier is the bile salt/acid stimulating substance. In another embodiment, the bile salt/acid stimulating substance is a substance separate from the carrier. Each possibility represents a separate embodiment of the present invention.

In another embodiment, an oil component of the present invention contains a significant quantity of one or more anti-oxidants. For example, sea buckthorn (oblepicha) oil contains a significant quantity of beta-carotene. In another embodiment, any other oil enriched in one or more anti-oxidants may be used. Each possibility represents a separate

embodiment of the present invention.

In another embodiment, an oil component of the present invention comprises a component that has a melting temperature ($T_m$) of at least 10°C. In another embodiment, the high $T_m$ component is an oil. In another embodiment, the carrier is the high $T_m$ component. In another embodiment, the high-$T_m$ component is included in addition to the carrier. An example of a high-$T_m$ oil is jojoba oil. In another embodiment, the high $T_m$ oil is any other high melting temperature oil known in the art. In another embodiment, the high $T_m$ oil is used as the oil carrier in the first oil component of a matrix carrier of the present invention. Each possibility represents a separate embodiment of the present invention.

In another embodiment, a matrix composition of the present invention further comprises a third oil or mixture of oils. In another embodiment, the third oil component comprises an antioxidant. In another embodiment, the third oil component is sesame oil. In another embodiment, the third oil component is another suitable oil known in the art. In another embodiment, the third oil, oil or mixture of oils has a higher viscosity than the additional oil or mixture of oils. Each possibility represents a separate embodiment of the present invention.

In another embodiment, a matrix composition of the present invention further comprises an additional oil component. As provided herein, mixing of multiple oil components of compositions of the present invention in the correct order provides self-ordering or self-organization of matrix structure, due to competitive adsorption and minimization of the free energy. The term "additional oil component" encompasses an oil or mixture of oils, as described elsewhere herein. In another embodiment, the oil carrier of the additional oil component is olive oil. In another embodiment, the oil carrier is another suitable oil known in the art. In another embodiment, the additional oil component comprises an antioxidant. Each possibility represents a separate embodiment of the present invention.

In another embodiment, the insulin protein is included in the additional oil or mixture of oils, instead of in the first-added oil or mixture of oils. In another embodiment, the insulin protein is combined with an antioxidant and oil (the first-added or additional oil or mixture of oils) prior to adding to the solid phase. Each possibility represents a separate embodiment of the present invention.

In another embodiment, the additional oil, oil or mixture of oils has a higher viscosity than the first-added oil or mixture of oils. In another embodiment, without wishing to be bound by any theory or mechanism of action, the use of a higher viscosity oil or oil mixture at this stage enables self-ordering or self-organization of structure due to competitive adsorption and minimization of the free energy. Each possibility represents a separate embodiment of the present invention.

In another embodiment, a matrix composition of the present invention further comprises a third oil or mixture of oils. In another embodiment, the third oil component comprises an antioxidant. In another embodiment, the oil carrier of the third oil component is sesame oil. In another embodiment, the oil carrier is another suitable oil known in the art. In another embodiment, the third oil, oil or mixture of oils has a higher viscosity than the additional oil or mixture of oils. Each possibility represents a separate embodiment of the present invention.

In another embodiment, a highly penetrative oil carrier is included in the outer oil or mixture of oils. Examples of highly penetrative oils are sesame oil, tea tree (Melaleuca) oil, lavender oil, almond oil, and grape seed oil. In another embodiment, the highly penetrative oil carrier promotes efficient transport of the substances into the blood. Each possibility represents a separate embodiment of the present invention.

In another embodiment, a matrix composition or pharmaceutical composition of the present invention further comprises a pharmaceutically acceptable wax. The term "wax" means a lipophilic compound, which is solid at room temperature (25 °C), with a reversible solid/liquid change of state, having a melting point of greater than or equal to 30 °C, which may be up to 120 °C. By bringing the wax to the liquid state (melting), it is possible to render it miscible with any oils present and to form a microscopically homogeneous mixture, but on returning the temperature of the mixture to room temperature, recrystallization of the wax in the oils of the mixture is obtained. The wax may be a natural wax, for example bees wax, a wax derived from plant material, or a synthetic wax prepared by esterification of a fatty acid and a long chain alcohol. Other suitable waxes include petroleum waxes such as a paraffin wax. In another embodiment, the wax stabilizes the matrix carrier composition. In another embodiment, the inclusion of wax facilitates formation of a tablet containing the matrix carrier composition. Each possibility represents a separate embodiment of the present invention.

## Insulin proteins

"Insulin protein" as used herein includes rapid-acting insulin, very rapid-acting insulin, intermediate-acting insulin, and long-acting insulin. Examples of rapid-acting insulin are lyspro insulin (Lysine-Proline insulin, sold by Eli Lilly as Humalog™), glulysine insulin (sold by Sanofi-Aventis as Apidra™), Actrapid™ and NovoRapid™ (both available from Novo Nordisk), aspart insulin (sold by Novo Nordisk as Novolog™). An example of very rapid-acting insulin is Viaject™, marketed by Biodel. Examples of intermediate-acting insulin are NPH (Neutral Protamine Hagedorn) and Lente insulin. An example of long-acting insulin is Lantus™ (insulin glargine). In some preferred embodiments, the insulin is Insugen™ from Biocon™. In another embodiment, the insulin is a mixture of different types of insulin. Some Examples of a such

a mixture are Mixtard® 30, Mixtard® 40, and Mixtard® 50, which are mixtures of different proportions of short-acting insulin and NPH (intermediate duration) insulin. In another embodiment, the insulin is any other type of insulin known in the art. In another embodiment, the insulin is naturally occurring insulin. In another embodiment, the insulin is a modified form of insulin. It will be clear from the present disclosure that use and compositions of the present invention are suitable for every type of natural and modified insulin known in the art. Each possibility represents a separate embodiment of the present invention.

**Additional components**

In another embodiment, the composition further comprises an antioxidant. In another embodiment, the antioxidant is a pharmaceutically acceptable antioxidant. In another embodiment, the antioxidant is selected from the group consisting of vitamin E, superoxide dismutase (SOD), omega-3, and beta-carotene. Each possibility represents a separate embodiment of the present invention.

In another embodiment, the composition further comprises an enhancer of the insulin protein. Examples of insulin enhancers include: dodecylmaltoside, octylglucoside, and dioctyl sodium sulphosuccinate. In another embodiment, the composition further comprises a cofactor of the insulin protein. An example of an insulin cofactor is chromium. Each possibility represents a separate embodiment of the present invention.

In another embodiment, a composition of the present invention further comprises a glucagon-like peptide or glucagon-like peptide analogue. Glucagon-like peptides and their analogues are well known in the art, and are described, *inter alia,* in Eleftheriadou I. et al. (The effects of medications used for the management of diabetes and obesity on postprandial lipid metabolism. Curr Diabetes Rev 4(4):340-56, 2008 and Vaidya HB et al., Glucagon like peptides-1 modulators as newer target for diabetes. Curr. Drug Targets 9(10):911-20, 2008). Each possibility represents a separate embodiment of the present invention.

In another embodiment, a composition of the present invention further comprises a bioflavonoid. Bioflavonoids are well known in the art, and are described, *inter alia,* in Ververidis F. et al. (Biotechnology of flavonoids and other phenylpropanoid-derived natural products. Part I: Chemical diversity, impacts on plant biology and human health and Part II: Reconstruction of multienzyme pathways in plants and microbes, 2(10):1214-49, 2007). Each possibility represents a separate embodiment of the present invention.

In another embodiment, a composition of the present invention further comprises a pharmaceutical-grade surfactant. Surfactants are well known in the art, and are described, *inter alia,* in the Handbook of Pharmaceutical Excipients (eds. Raymond C. Rowe, Paul J. Sheskey, and Sian C. Owen, copyright Pharmaceutical Press, 2005). In another embodiment, the surfactant is any other surfactant known in the art. Each possibility represents a separate embodiment of the present invention.

In another embodiment, a composition of the present invention further comprises pharmaceutical-grade emulsifier or emulgator (emollient). Emulsifiers and emulgators are well known in the art, and are described, *inter alia,* in the Handbook of Pharmaceutical Excipients (ibid). Examples of emulsifiers and emulgators are eumulgin, Eumulgin B 1 PH, Eumulgin B2 PH, hydrogenated castor oil cetostearyl alcohol, and cetyl alcohol. In another embodiment, the emulsifier or emulgator is any other emulsifier or emulgator known in the art. Each possibility represents a separate embodiment of the present invention.

In another embodiment, a composition of the present invention further comprises pharmaceutical-grade stabilizer. Stabilizers are well known in the art, and are described, *inter alia,* in the Handbook of Pharmaceutical Excipients (ibid). In another embodiment, the stabilizer is any other stabilizer known in the art. Each possibility represents a separate embodiment of the present invention.

In another embodiment, the weight of the particulate matter of a composition of the present invention is not more than 33% of the weight of the oil phase. The particulate matter is composed of the silica nanoparticles, the branched polysaccharide, the insulin, and any other solid components that may be incorporated into the matrix. In another embodiment, the particulate matter is composed of the silica nanoparticles, the branched polysaccharide, and the insulin. The weight of the particulate matter is the weight of the oil carrier plus additional oils mixed therewith and substances dissolved therein, if any, for all the oil components combined. In another embodiment, the weight of the particulate matter is not more than 30% of the weight of the oil phase. In another embodiment, the weight of the particulate matter is not more than 25% of the weight of the oil phase. In another embodiment, the weight of the particulate matter is not more than 20% of the weight of the oil phase. In another embodiment, the weight of the particulate matter is not more than 15% of the weight of the oil phase. In another embodiment, the weight of the particulate matter is not more than 10% of the weight of the oil phase. In another embodiment, the weight of the particulate matter is not more than 8% of the weight of the oil phase. In another embodiment, the weight of the particulate matter is not more than 5% of the weight of the oil phase. Each possibility represents a separate embodiment of the present invention.

In another embodiment, the weight of the particulate matter is not more than 75% of the total weight of the composition. In another embodiment, the weight of the particulate matter is not more than 50% of the total weight of the

composition. In another embodiment, the weight of the particulate matter is not more than 25% of the total weight of the composition. In another embodiment, the weight of the particulate matter is not more than 30% of the total weight of the composition. In another embodiment, the weight of the particulate matter is not more than 20% of the total weight of the composition. In another embodiment, the weight of the particulate matter is not more than 15% of the total weight of the composition. In another embodiment, the weight of the particulate matter is not more than 10% of the total weight of the composition. In another embodiment, the weight of the particulate matter is not more than 8% of the total weight of the composition. In another embodiment, the weight of the particulate matter is not more than 6% of the total weight of the composition. In another embodiment, the weight of the particulate matter is not more than 5% of the total weight of the composition. Each possibility represents a separate embodiment of the present invention.

**Administration**

In another embodiment, the present invention provides the use of the pharmaceutical composition for orally administering insulin to a subject.

**Formulation methods**

In another embodiment, the present invention provides a method of manufacturing a pharmaceutical composition for oral delivery of insulin, the method comprising the steps of: (a) dry blending pharmacologically inert silica nanoparticles having a hydrophobic surface, wherein the size of the silica nanoparticles is between 1-100 nanometers, with at least one branched polysaccharide, whereby the silica nanoparticles form an intimate non-covalent association with the at least one branched polysaccharide; (b) mixing or dissolving an insulin protein into an oil; and (c) mixing the silica nanoparticles and at least one branched polysaccharide into the oil, wherein the silica nanoparticles, at least one branched polysaccharide, and insulin are suspended in, embedded in or dispersed in the oil. Preferably, the silica nanoparticles and at least one branched polysaccharide form a complex. In another embodiment, the complex is suspended in, embedded in or dispersed in the oil. In another embodiment, the insulin protein is attached to the hydrophobic surfaces of the silica nanoparticles and the at least one branched polysaccharide via non-covalent forces. In another embodiment, the particle size of the matrix carrier composition is between 100-500,000 nanometers. In some preferred embodiments, the particle size is between 100-50,000 nanometers. In another embodiment, the particle size is between 100-5,000 nm. Each possibility represents a separate embodiment of the present invention.

Formulation methods of the present invention encompass embodiments wherein additional components are present in step (a). In another embodiment, more than one type of biopolymer is present together with the silica nanoparticles. In another embodiment, a branched polysaccharide and a dietary fiber are present together with the silica nanoparticles. In another embodiment, a branched polysaccharide and a linear polysaccharide are present together with the silica nanoparticles. In another embodiment, a branched biopolymer, a linear polysaccharide, and an insoluble fiber are present together with the silica nanoparticles.

In another embodiment, the present invention provides a method of manufacturing a pharmaceutical composition for oral delivery of insulin, the method comprising the steps of: (a) blending pharmacologically inert silica nanoparticles having a hydrophobic surface, wherein the size of the silica nanoparticles is between 1-100 nanometers, with (i) at least one branched polysaccharide and (ii) an insulin protein whereby the silica nanoparticles form an intimate non-covalent association with the at least one branched polysaccharide; and (b) mixing the silica nanoparticles, at least one branched polysaccharide, and insulin protein into an oil. In certain embodiments, the insulin protein in the form of a dry lyophilized powder is directly dissolved into the oil of step (b). Preferably, the silica nanoparticles, at least one branched polysaccharide, and insulin form a complex. In another embodiment, the silica nanoparticles, branched polysaccharide, and insulin form a matrix that becomes suspended in, embedded in or dispersed in the oil. In another embodiment, the insulin protein is non-covalently attached to the hydrophobic surfaces of the silica nanoparticles and the at least one branched polysaccharide. In another embodiment, the particle size of the pharmaceutical composition is between 100-500,000 nanometers. In some preferred embodiments, the particle size is between 100-50,000 nanometers. In other embodiments, the particle size is between 100-5,000 nanometers. Each possibility represents a separate embodiment of the present invention.

In another embodiment, the insulin is extracted from an aqueous solution. In another embodiment, an aqueous insulin solution is mixed with oil, resulting in extraction or dispersion of the insulin directly into the oil phase of the resulting emulsion. Methods for extracting active enzymes such as insulin from an aqueous solution are well known in the art. In another embodiment, a gel-forming water phase stabilizer is used to extract the insulin. An example of a gel-forming water phase stabilizer is Silica 380, which is available as pharma-grade hydrophilic nanoparticles. Each possibility represents a separate embodiment of the present invention.

In another embodiment, step (b) of the above method comprises the step of directly dissolving or dispersing a lyophilized protein into the oil or oil mixture. In another embodiment, a solution of the insulin protein is mixed with the

oil or oil mixture and the aqueous phase is then removed. In another embodiment, a solution of the insulin protein is mixed with the oil or oil mixture forming water-in-oil emulsion. Each possibility represents a separate embodiment of the present invention.

The properties and classification of the silica nanoparticles, branched polysaccharide, and insulin protein of the above methods may be any of those described herein. Each possibility represents a separate embodiment of the present invention. "Oil" as referred to in methods of the present invention can refer to either a single oil, a mixture of oils, or an oil phase. As described herein, a mixture of oils or oil phase will typically comprise an oil carrier. In another embodiment, the mixture of oils or oil phase further comprises an additional oil or oils or an additional component or components. Each possibility represents a separate embodiment of the present invention.

In another embodiment, step (a) of a method of manufacturing a pharmaceutical composition for oral delivery of insulin of the present invention further comprises the step of confirming that the silica nanoparticles and branched polysaccharide are properly homogenized. In another embodiment, any of the following three tests are utilized: (a) the mixture appears homogenous; (b) the volume of the mixture is smaller than the sum of volumes of the 2 components; and (c) the mixture does not sink when placed on the surface of a still body of water. In another embodiment, the composition reaches a minimum volume that is not decreased upon further mixing. Each possibility represents a separate embodiment of the present invention.

The step of dry mixing is, in another embodiment, performed using a high shear mixer. In another embodiment, the step of mixing is performed using a high-speed mixer. In another embodiment, the step of mixing is performed using any other means suitable for generating a homogenous solid phase from silica nanoparticles and a branched polysaccharide. Each possibility represents a separate embodiment of the present invention.

In another embodiment, step (a) of a method of manufacturing a pharmaceutical composition for oral delivery of insulin of the present invention, i.e. the dry mixing step, further comprises inclusion of an additional biopolymer that is a linear biopolymer. In another embodiment, the additional biopolymer is a linear polysaccharide. In another embodiment, the additional biopolymer is a linear high molecular weight structural protein. In another embodiment, the additional biopolymer is selected from the group consisting of chitin, cellulose, a linear alpha glucan, and a linear beta glucan. In another embodiment, the additional biopolymer is selected from the group consisting of chitin, amylose, cellulose, and beta glucan. In another embodiment, a formulation method of the present invention comprises the steps of including in the solid phase mixture both a branched polysaccharide and a linear polysaccharide. Formulation methods are provided herein that comprise inclusion of amylopectin, a branched polysaccharide, and chitin, a linear polysaccharide (Example 5). Other branched polysaccharides and linear polysaccharides disclosed herein are suitable as well. Each possibility represents a separate embodiment of the present invention.

In another embodiment, the additional biopolymer of formulation methods of the present invention is a dietary fiber, also known as "roughage." In another embodiment, the dietary fiber is an insoluble fiber. In another embodiment, the dietary fiber is a linear insoluble fiber. In another embodiment, the dietary fiber is a soluble fiber. In another embodiment, the dietary fiber is a linear soluble fiber.

In another embodiment, a method of manufacturing a pharmaceutical composition for oral delivery of insulin of the present invention comprises the steps of including a branched biopolymer, a linear polysaccharide, and an insoluble fiber. In another embodiment, the method comprises the steps of including in the particulate matter mixture a branched polysaccharide, a linear polysaccharide, and an insoluble fiber. An example of such is a composition comprising amylopectin, a branched polysaccharide; chitin, a linear polysaccharide; and cellulose, an insoluble fiber. Other branched and linear polysaccharides and insoluble fibers disclosed herein are suitable as well. Each possibility represents a separate embodiment of the present invention.

In another embodiment, a method of manufacturing a pharmaceutical composition for oral delivery of insulin of the present invention further comprises the step of adding an additional oil following the addition of the first-added oil or mixture of oils. The term "additional oil" encompasses an oil or mixture of oils, as described elsewhere herein. In another embodiment, the additional oil component comprises an antioxidant. Each possibility represents a separate embodiment of the present invention.

In another embodiment, the insulin protein is included in the additional oil or mixture of oils, instead of in the first-added oil or mixture of oils.

In another embodiment, the additional oil, oil or mixture of oils has a higher viscosity than the first-added oil or mixture of oils. In another embodiment, the use of a higher viscosity oil or oil mixture at this stage enables formation of ordered structures in the composition.

In another embodiment, a method of manufacturing a pharmaceutical composition for oral delivery of insulin of the present invention further comprises the step of adding a third oil or mixture of oils after addition of the above-described additional oil or mixture of oils. In another embodiment, the third oil component comprises an antioxidant. Each possibility represents a separate embodiment of the present invention.

In another embodiment, a highly penetrative oil carrier is included in the outer oil or mixture of oils. In another embodiment, the highly penetrative oil carrier promotes efficient transport of the substances into the blood. Each possibility

represents a separate embodiment of the present invention.

In another embodiment, a method of manufacturing a pharmaceutical composition for oral delivery of insulin of the present invention further comprises the step of adding a pharmaceutically acceptable wax following the addition of the first-added oil or mixture of oils. In another embodiment, the wax is a substance with properties similar to beeswax. In another embodiment, the wax is a substance having the following properties: (a) plastic (malleable) at normal ambient temperature; (b) having a melting point above approximately 45 °C (113 °F); (c) a low viscosity when melted, relative to a typical plastics; (d) insoluble in water; and (e) hydrophobic. In certain preferred embodiments, the wax is beeswax. In another embodiment, the wax stabilizes the matrix carrier composition. In another embodiment, the inclusion of wax facilitates formation of a tablet containing the matrix carrier composition. Each possibility represents a separate embodiment of the present invention.

In another embodiment, the wax is heated as part of a method of the present invention. In another embodiment, the wax is pulverized. In another embodiment, the wax is both heated and pulverized. In another embodiment, the heating and/or pulverization are performed prior to blending with the other components. In another embodiment, the wax remains hot while blending with the other components is begun. In another embodiment, the heating and/or pulverization are performed during blending with the other components. In another embodiment, the heating and/or pulverization are performed both prior to and during blending with the other components. Each possibility represents a separate embodiment of the present invention.

In another embodiment, a composition of the present invention further comprises one or more pharmaceutically acceptable excipients, into which the matrix carrier composition is mixed. In another embodiment, the excipients include one or more additional polysaccharides. In another embodiment, the excipients include one or more waxes. In another embodiment, the excipients provide a desired taste to the composition. In another embodiment, the excipients influence the drug consistency, and the final dosage form such as a gel capsule or a hard gelatin capsule.

Examples of excipients include: Antifoaming agents (dimethicone, simethicone); Antimicrobial preservatives (benzalkonium chloride, benzelthonium chloride, butylparaben, cetylpyridinium chloride, chlorobutanol, chlorocresol, cresol, ethylparaben, methylparaben, methylparaben sodium, phenol, phenylethyl alcohol, phenylmercuric acetate, phenylmercuric nitrate, potassium benzoate, potassium sorbate, propylparaben, propylparaben sodium, sodium benzoate, sodium dehydroacetate, sodium propionate, sorbic acid, thimerosal, thymol); Chelating agents (edetate disodium, ethylenediaminetetraacetic acid and salts, edetic acid); Coating agents (sodium carboxymethyl-cellulose, cellulose acetate, cellulose acetate phthalate, ethylcellulose, gelatin, pharmaceutical glaze, hydroxypropyl cellulose, hydroxypropyl methylcellulose, hydroxypropyl methylcellulose phthalate, methacrylic acid copolymer, methylcellulose, polyethylene glycol, polyvinyl acetate phthalate, shellac, sucrose, titanium dioxide, carnauba wax, microcrystalline wax, zein); Colorants (caramel, red, yellow, black or blends, ferric oxide); Complexing agents (ethylenediaminetetraacetic acid and salts (EDTA), edetic acid, gentisic acid ethanolmaide, oxyquinoline sulfate); Desiccants (calcium chloride, calcium sulfate, silicon dioxide); Emulsifying and/or solubilizing agents (acacia, cholesterol, diethanolamine (adjunct), glyceryl monostearate, lanolin alcohols, lecithin, mono- and di-glycerides, monoethanolamine (adjunct), oleic acid (adjunct), oleyl alcohol (stabilizer), poloxamer, polyoxyethylene 50 stearate, polyoxyl 35 caster oil, polyoxyl 40 hydrogenated castor oil, polyoxyl 10 oleyl ether, polyoxyl 20 cetostearyl ether, polyoxyl 40 stearate, polysorbate 20, polysorbate 40, polysorbate 60, polysorbate 80, propylene glycol diacetate, propylene glycol monostearate, sodium lauryl sulfate, sodium stearate, sorbitan monolaurate, sorbitan monooleate, sorbitan monopalmitate, sorbitan monostearate, stearic acid, trolamine, emulsifying wax); Flavors and perfumes (anethole, benzaldehyde, ethyl vanillin, menthol, methyl salicylate, monosodium glutamate, orange flower oil, peppermint, peppermint oil, peppermint spirit, rose oil, stronger rose water, thymol, tolu balsam tincture, vanilla, vanilla tincture, vanillin); Humectants (glycerin, hexylene glycol, propylene glycol, sorbitol); Polymers (e.g., cellulose acetate, alkyl celluloses, hydroxyalkylcelluloses, acrylic polymers and copolymers); Suspending and/or viscosity-increasing agents (acacia, agar, alginic acid, aluminum monostearate, bentonite, purified bentonite, magma bentonite, carbomer 934p, carboxymethylcellulose calcium, carboxymethylcellulose sodium, carboxymethycellulose sodium 12, carrageenan, microcrystalline and carboxymethylcellulose sodium cellulose, dextrin, gelatin, guar gum, hydroxyethyl cellulose, hydroxypropyl cellulose, hydroxypropyl methylcellulose, magnesium aluminum silicate, methylcellulose, pectin, polyethylene oxide, polyvinyl alcohol, povidone, propylene glycol alginate, silicon dioxide, colloidal silicon dioxide, sodium alginate, tragacanth, xanthan gum); Sweetening agents (aspartame, dextrates, dextrose, excipient dextrose, fructose, mannitol, saccharin, calcium saccharin, sodium saccharin, sorbitol, solution sorbitol, sucrose, compressible sugar, confectioner's sugar, syrup); This list is not meant to be exclusive, but instead merely representative of the classes of excipients and the particular excipients which may be used in oral dosage compositions of the present invention. Each possibility represents a separate embodiment of the present invention.

As provided herein, methods have been developed to formulate insulin in orally administrable form. The components are, in some preferred embodiments, mixed in a particular order in order to produce oil-coated matrix carrier compositions that protect the active ingredient from digestive processes in the stomach. Without wishing to be bound by any theory or mechanism of action, the biopolymer, particularly when branched, absorbs hydraulic and mechanical stresses experienced during digestion. The oil coating constitutes a physical barrier that provides additional protection

against digestive enzymes. Secretion of bile acids typically causes dispersion of the oil suspension into smaller particles, which can be absorbed in the small intestine. While the particle size is reduced after traversing the stomach and entering the small intestine, the particles remain in a size range of 30-1000 nm, too large to be a substrate for lipases and peptidases, preserving the protective effect of the composition. Advantageously, lipid-coating particles of this size are absorbed to chylomicrons by lacteal vessels, which are lymphatic vessels originating in the villi of the small intestine. Particles absorbed in this manner can reach the bloodstream without undergoing first-pass metabolism, largely preserving the biological activity of the insulin.

Matrix carriers for any insulin protein can be designed using the following principles:

For purposes of illustration, the following formulas may be utilized in practicing the invention:

1. Quantify the R972 hydrophobic silica (specific area is about $110 m^2 * g^{-1}$)

$$Si \geq 10 * \frac{D_{IU}}{27.5}$$

wherein: $D_{IU}$ is desired dosage of insulin per 1 ml in IU; and Si is the concentration of silica $mg*ml^{-1}$

Note: 27.5 $IU*mg^{-1}$ is the specific activity of regular insulin; the formula should be adjusted accordingly for other types of insulin.

2. The specific weight of silica is about 2.4 $g*cm^{-3}$, the concentration of the branched polysaccharide in the medication must therefore be at least 2.5 times of silica:

$$G_{AP} \geq 2.4 * Si$$

Wherein: $G_{AP}$ is the concentration of amylopectin.

3. The concentration of the linear polysaccharides ($G_{LP}$) is estimated as:

$$0.3 \leq \frac{G_{LP}}{G_{AP}} \leq 0.7$$

Note: Using the above mentioned concentration ratios between the silica nanoparticles and the polysaccharides, ensures the stability of the pharmaceutical composition.

4. The chitin/fiber ratio may be between 0-1. High ratios (>0.5) are used for the formation of fast term release insulin compositions.

5. Thickness of the protective oil layer is at least 10 times the diameter of a globular molecule or the maximal branch size of an elongated molecule. The thickness of the oil coating of the pharmaceutical compositions of the present invention is determined by the following properties of the oil or mixture of oils: (a) the viscosity and melting temperature; (b) the acidity; and (c) the concentration of polar groups. Typically, a first type of oil is chosen, preferably having a relatively low viscosity and low concentration of polar groups. Suitable examples are evening primrose oil, sesame oil, and silicon oil.

6. The concentration of insulin in the final formulation is determined, based on its pharmacokinetics and pharmacodynamics.

The principles of the present invention are demonstrated by means of the following examples.

## EXAMPLES

### EXAMPLE 1: Preparation of insulin composition

An insulin composition (Formulation I) was produced, using the following ingredients:

- Insulin Actrapid™, 9 ml

- Olive oil, 11 ml
- Benefiber™, 7 g
- Silica R972, 1.2 g
- Oblepicha, 9 ml
- Sesame Oil up to 75 ml

Insulin was combined with sea buckthorn (oblepicha) oil and stirred at 20 rpm for 2 min, and then at 50 rpm for 5 min. Benefiber™ (Novartis Nutrition GmbH, Germany) and hydrophobic silica R972 were placed into a beaker and mixed by vortexing at 900 rpm for 5 min. Association between the Benefiber ™ and the silica was determined by the mixture's ability to float after being placed on the surface of a water-filled beaker. The Benefiber™/silica mixture was added to the oil-insulin solution and stirred for 25 minutes at 50 rpm. Olive oil was added, and the mixture was stirred at 50 rpm for 3 min. The volume was brought up to 75 ml with sesame oil, and the mixture was stirred at 50 rpm for 20 min. The product was stored refrigerated (3-8 °C). The final insulin concentration was 12 IU/ml. For animal experiments, the composition was administered by gavage. Other preparation of the product were packaged into gelatin enteric covering capsules.

**EXAMPLE 2: Additional Actrapid™ matrix carrier composition formulated for short-life**

An additional Actrapid™ formulation (Formulation II) using the following ingredients was designed for short-term insulin release:

- Insulin Actrapid™, 1 ml
- Olive oil, 1.5 ml.
- Ambrotose™, 0.7 g.
- Silica R972, 0.1 g
- Oblepicha oil, 1.5 ml
- Evening primrose oil, 5 ml

0.7 g of rice polysaccharides (Ambrotose™, Mannatech Inc, Coppell, TX 75019, USA) was combined with 0.1 g hydrophobic fumed silica R972 (Degussa Inc), and mixed by vortexing at 900 rpm for 5 min. Association between the Ambrotose™ and the silica was determined by the mixture's ability to float after being placed on the surface of a water-filled beaker 1 ml Actrapid™ insulin were added and stirred for 15 minutes at 50 rpm. 1.5 ml of olive oil was added and stirred for 2 minutes at 100 rpm with a magnetic stirrer. Sea buckthorn (oblepicha) oil was added and stirred for 2 minutes at 100 rpm with a magnetic stirrer. The volume was brought up to 5 ml with evening primrose oil and stirred at 50 rpm for 20 min. The product was stored refrigerated (3-8 °C). In a separate preparation, the amount of ingredients used was doubled, yielding identical results.

The final insulin concentration was 20 IU/ml. For human administration, the product was packaged into 25 gelatin enteric covering capsules.

In additional experiments, vitamin E was included in any one of the oils used.

**EXAMPLE 3: Longterm release Actrapid™ matrix carrier composition**

The following formulation (Formulation III) was manufactured to provide longer-term Actrapid™ release:

- Olive oil, 10 ml.
- Benefiber™, 1.5 g.
- Insulin Actrapid™, 2 ml

- Silica R972, 0.7 g
- Oblepicha oil, 10 ml
- Evening primrose oil, 5 ml
- Linseed oil, up to 40 ml.

Benefiber™ was combined with hydrophobic fumed silica R972 and mixed by vortexing at 900 rpm for 5 minutes. Association between the Benefiber™ and the silica was determined by the mixture's ability to float after being placed on the surface of a water-filled beaker. Actrapid™ insulin was added and stirred for 15 minutes at 50 rpm. Evening primrose oil was added and stirred for 2 minutes at 100 rpm with a magnetic stirrer. Sea buckthorn (oblepicha) oil was added and stirred for 2 minutes at 100 rpm with a magnetic stirrer. Olive oil was added and stirred for 2 minutes at 100 rpm with a magnetic stirrer. The volume was brought up to 40 ml with olive oil and stirred at 50 rpm for another 20 min. The product was stored refrigerated (3-8 °C).

The final insulin concentration was 5 IU/ml. For human administration, the product was packaged into 25 gelatin enteric covering capsules (commercially available from Shionogi and Company, Ltd, Japan) containing 1.6 ml = 7.8 IU insulin each.

**EXAMPLE 4: Preparation of additional Actrapid™ matrix carrier composition**

An additional Actrapid™ matrix carrier composition (Formulation V) was produced, using the following ingredients:

- Olive oil, 11 ml
- Benefiber™, 3 g
- Insulin Actrapid™, 9 ml
- Oblepicha oil, 9 ml
- Hydrophobic silica R972, 1.2 g
- Sesame Oil up to 75 ml.

Benefiber™ (Novartis Nutrition GmbH, Germany) and silica were placed into a beaker and mixed by vortexing at 900 rpm for 5 minutes. Association between the Benefiber ™ and the silica was determined by the mixture's ability to float after being placed on the surface of a water-filled beaker. Actrapid™ insulin was added and stirred for 15 minutes at 50 rpm. Sesame oil and sea buckthorn (oblepicha) oil were combined in a beaker and vortexed on a low setting for 15 minutes. Olive oil was added to the oils and stirred with a glass rod. The solid phase mixture and oil mixture were combined and mixed at 100 rpm with a magnetic stirrer. The volume was brought up to 75 ml with sesame oil and stirred with a glass rod. The product contained 12 IU/ml insulin and was packaged into gelatin enteric covering capsules.

An additional insulin matrix carrier composition (Formulation VI) was prepared using NovoRapid™ insulin, using the above protocol. In this case NovoRapid™ insulin was used instead of Actrapid™ insulin.

**EXAMPLE 5: Additional insulin matrix carrier composition:**

An additional insulin matrix carrier composition (Formulation IV) was prepared using BIOCON insulin, using the following protocol and the ingredients set forth in Table 1, using methods similar to those set forth in previous Examples. A light microscopy picture of the composition is shown in Figure 3.

1. Mix oblepicha + olive oil + 1/3 of sesame oil.
2. Add Insugen™ insulin powder (BIOCON) into the mixture of oils and mix.
3. Mix fiber + chitin + amylopectin + silica.

4. Add the mixture of step 3 to the mixture of oils and insulin of step 2 and mix.

5. Add the rest of the sesame oil and mix.

Table 1. Ingredients for the preparation of the BIOCON matrix carrier composition.

| **Formulation IV** | | | |
|---|---|---|---|
| Insulin Powder (BIOCON), mg | 36.4 | 109.2 | 182 |
| Olive Oil, ml | 33 | 33 | 33 |
| Sea Buckthorn Oil (Oblepicha), ml | 42 | 42 | 42 |
| Sesame Oil, ml | up to 100 | up to 100 | up to 100 |
| Amylopectin, g | 11.25 | 11.25 | 11.25 |
| Chitin, g | 1.9 | 1.9 | 1.9 |
| Silica R972, g | 2.5 | 2.5 | 2.5 |
| Final concentration of insulin, IU/ml | **10** | **30** | **50** |

Next, an additional short-term release insulin matrix carrier composition (Formulation A) was prepared using BIOCON insulin, using the ingredients set forth in Table 2.

Table 2. Ingredients for the preparation of an insulin matrix carrier composition (Formulation A).

| **Ingredient** | **Amount** |
|---|---|
| Olive oil | 20 ml |
| Ambrotose* | 3g |
| Insulin powder | 70 mg |
| Silica R972 | 0.6g |
| Sea buckthorn (oblepicha) oil | 26ml |
| Sesame oil up to | 70ml |

Ambrotose™ powder contains Arabinogalactan (a gum from the Larix decidua tree), Manapol, which is a gel extracted from the inner leaf of aloe vera gel plant, gum ghatti, and gum tragacanth or Manapol powder, oat fiber, brown macroalgae (Undaria pinnatifida) sporophyll, vegetarian glucosamine-HCl, ghatti gum, gum tragacanth and xylitol.

**EXAMPLE 6: Efficacy of the oral insulin composition of the present invention in diabetic mice:**

**MATERIALS AND EXPERIMENTAL METHODS**

**Streptozotocin (STZ)-induced diabetes treatment:** Diabetes was induced by 2 injections of 500 and 700 μl of 1.5 mg/ml streptozotocinseparated by 48 hr, in male adult BALB/c mice (7-10 weeks old) of an average weight of 23-28 gr. Untreated mice of approximately the same age and weight were used as control. Blood glucose levels (BGL) were assessed 48 hours after STZ injection by a standard FreeStyle™ glucometer (Abbot Diabetes Cere Inc, Alameda, CA) from the tail vein blood samples.

Insulin compositions were administered orally to mice by gavage (1 ml volume), without prior deprivation of food or water. During the experiment mice were supplied with food and water as usual.

**Compositions:** The first experiment utilized Formulations V and VI described in Example 4. The second experiment utilized Formulation IV described in Example 5.

**Blood insulin concentration.** Blood insulin concentrations were detected by ELISA (Human Insulin ELISA kit, Linco).

**Treatment groups:**

**1.** Control group 1: no STZ treatment, no insulin administration.

2. Control group 2: no STZ treatment, oral insulin composition of the present invention administered by gavage

3. Control group 3: STZ treated, no insulin administration.

4. Diabetic (STZ treated) mice; insulin administered by SC injection.

5. Diabetic (STZ treated) mice; insulin was administered by gavage.

6. Diabetic (STZ treated) mice; insulin was administered using the oral insulin composition of the present invention by gavage.

7. Diabetic (STZ treated) mice; matrix carrier (without insulin) administered by gavage as control.

**RESULTS**

In a first experiment, diabetes was induced by streptozotocin (STZ) in male adult BALB/c mice, followed by administration of NovoRapid™ (9.5 IU) and Actrapid™ (12 IU) based insulin compositions of the present invention. Both compositions significantly reduced blood glucose levels (Figures 4A-B, respectively). By contrast, STZ-treated mice that received empty matrix carrier compositions (lacking insulin), orally administered Actrapid™ or NovoRapid™ insulin, or were given 25 IU Insulin (BIOCON) in PBS (gavage) (Figure 5) did not exhibit significant reduction in blood glucose levels. Normal mice (not STZ-treated) that received insulin compositions exhibited no significant reduction in blood glucose level. Normal and diabetic mice injected with insulin, by contrast, exhibited hypoglycemia symptoms that were in some cases fatal.

In a second experiment, an insulin composition of the present invention was administered orally to STZ-treated mice by gavage (1 ml volume) in dosages ranging from 2-10 IU. A dose-responsive reduction in blood glucose levels was observed for 9-12 hours; however, levels rarely dropped below 100 mg/dL (Figure 6A-D). The presence of human insulin in the blood following administration of the insulin matrix carrier composition was confirmed by ELISA. By contrast, subcutaneous injection of 10 IU of insulin caused near-fatal hypoglycemia (Figure 6E). Normal mice receiving 2, 5, or 10 IU insulin compositions exhibited only a slight reduction in blood glucose level (Figure 6F), while those receiving injected insulin experienced a precipitous and occasionally fatal drop in glucose levels. As before, STZ-treated mice that received empty matrix carrier compositions (lacking insulin), orally administered insulin, or were left untreated did not exhibit significant blood glucose level reduction.

In another experiment, direct comparison of 10, 5, or 2 IU of insulin matrix carrier composition (Formulation IV) vs. injection of the same amount of insulin solution (a standard formulation) in 14-25 g mice reveled that mice treated with the oral insulin composition of the present invention maintained normal blood glucose levels for longer periods of time compared to the insulin injected mice. These observation reflect on the increased bioavailability of insulin when administered within the matrix carrier composition of the present invention. In addition, mice administered the matrix carrier compositions had no hypoglycemia, while the injected mice exhibited severe hypoglycemia (Figures 6G, H, I for 10, 5, and 2 IU, respectively).

Figures 6J-K: We have compared the pharmacodynamics of Formulation A (Example 5) and. Formulation IV (Example 5). Results are described in figure 6J (2 IU of insulin) and figure 6J (7.5 IU of insulin) according to which mice administered Formulation IV showed lower blood glucose levels for longer periods of time as opposed to mice administered Formulation A.

Indication for the increased bioavailability of the insulin administered orally using the compositions of the present invention in comparison with injected insulin can be found by calculating the "Effective Areas". "Effective Area" is defined as the sum of the net changes in blood glucose level (BGL) values relative to the basal level, along a defined period of time, calculated as follows:

1. Obtain a baseline average of BGL for each time point.

2. For each time point, subtract the BGL value in the treated groups (oral insulin of the present invention and injected insulin) from the baseline average.

3. Sum the values obtained in step 2 for all time points.

To obtain the values in the table, the "effective areas" of the different treatments were then subtracted or divided. Results are depicted in Table 3.

Table 3. Effective Areas of insulin oral matrix carrier compositions versus injected insulin.

| Group | Value |
|---|---|
| 10 IU: (injection- matrix carrier composition) | -1042.7 mg/dL |
| 5 IU: (injection- matrix carrier composition) | 340.29 mg/dL |
| 2 IU: (injection- matrix carrier composition) | 834.4 mg/dL |
| | |
| 10IU: injection / matrix carrier composition | 0.64 |
| 5IU: injection / matrix carrier composition | 1.32 |
| 2IU: injection / matrix carrier composition | 3.73 |
| | |
| 10IU injection / 5 IU injection | 1.32 |
| 10IU injection / 2 IU injection | 1.61 |
| | |
| 10IU matrix carrier composition / 5 IU matrix carrier composition | 2.74 |
| 10 IU matrix carrier composition / 2 IU matrix carrier composition | 9.45 |

As shown in Table 2, the relatively low 10IU/5IU and 10IU/2IU ratios for injected insulin indicate that these doses are approaching the saturation dose for the mice. By contrast, the relatively large ratios for the insulin matrix carrier composition indicate that it is far from the saturation doses. Thus, matrix carrier compositions of the present invention are more amenable to accurate dosing within their therapeutic range, compared with standard injected insulin formulations.

**EXAMPLE 7: The efficacy of the oral insulin compositions of the present invention in human subjects:**

The effect of an insulin composition of the present invention was tested on two human subjects, one healthy and one diabetic. 30 IU of Actrapid™ matrix carrier composition (Formulation II) reduced blood glucose levels in the healthy subject from 105 to 80-90 mg/dL over a six-hour test period (Figure 7A). The subject reported an unusual degree of hunger, but otherwise no adverse reactions. By contrast, administration of injected insulin to healthy subjects is known to cause hypoglycemia, in some cases severe, with accompanying adverse reactions.

10 IU of the same formulation V (Example 4) were administered 3 times per day over 14 days to a 67-year-old subject having type I/II diabetes, who exhibited glucose levels of over 170 when untreated and 130-170 when receiving Gluco-Rite™. Upon taking the oral insulin composition of the present invention the blood glucose levels dropped to an average of about 130 (Figure 7B). The subject reported feeling well during the entire period of receiving the oral insulin composition of the present invention. The subject has continued to take the compositions 3-4 times per day, as needed, resulting in well-controlled glucose levels with no adverse reactions. By contrast, the subject had a long-standing history of intense sensations of dread and unease after receiving a number of different injected insulin formulations.

The presence of elevated insulin levels in the diabetic subject's blood following administration of the insulin composition was confirmed by ELISA.

Thus, insulin compositions of the present invention are capable of orally delivering various forms of insulin in a biologically active form that can effectively treat diabetes. They have the additional advantage of not inducing hypoglycemia in either diabetic or normal subjects.

**EXAMPLE 8: Toxicity study of chronic oral administration of insulin compositions:**

**MATERIALS AND EXPERIMENTAL METHODS**

Fifteen 10 week-old Balb/C male mice were used. Mice were administered daily 1ml of insulin matrix carrier composition (25IU/ml) (experimental group) or PBS (gavage control group) via gavage over 15 days. On the 14th and 15th day, mice were administered orally 100ng of lipopolysaccharides (LPS) together with the insulin matrix carrier composition or PBS. Negative control mice were administered 1ml PBS by gavage over 13 days, and 100ng of LPS in 1ml PBS by gavage on the 14th and 15th day. Positive control mice were injected with 1mg of LPS in 1ml of PBS. 3h after LPS administration, mice were sacrificed, blood was collected (for LPS detection) and gastro-system, liver and kidneys were fixed in paraformaldehyde (PFA) 4% for histological analysis.

**Animal follow up and macroscopic analysis:** Mice were weighed every 3 days, and their fur condition was detected daily. After sacrifice, all organs or tissues were investigated for the presence of pathological changes.

**Internal organs collection and fixation:** On day 15 mice were sacrificed, and their gastro-system, kidney, and liver were collected from the abdominal cavity, weighed and fixed in 10% formalin solution.

**Blood collection and plasma preparation:** Blood was collected from the heart of the mice into tubes that contain EDTA. Plasma was be separated from the blood by centrifugation; at first for 15 min at 3000×gmax followed by 15 min at 16,000×gmax. Supernatant was removed, placed in a new tube and stored at -20°C.

Detection of LPS in mouse serum: LPS in mouse serum was detected by HPLC. Sera taken on day 15 from the groups described above were prepared for HPLC analysis by addition of 0.1M of EDTA.

**RESULTS**

The toxicity of chronic administration of oral insulin compositions of the present invention was investigated. No pathological changes were observed in the animals' behavior. Their fur was in normal condition, appearing smooth, clean, and bright. No weight loss was detected; the mice gained weight normally.

Microscopic and macroscopic organ analysis:

Microscopic analysis showed no evidence of pathology in tissues of mice in all groups (liver- Figure 8A; kidney- Figure 8B; duodenum- Figure 8C). In addition, macroscopic analysis of internal organs revealed no evidence of any pathology. Organs of all mice were normally developed, had normal size, shape, appearance (bright and smooth), and weight, were normally colored, and were in their normal location. Livers were in all cases situated under diaphragm and smooth and bright, and weights were about 1.6 g. Liver parenchyma were dark colored. Kidneys exhibited a smooth surface. Weight was consistently about 0.2 gr. Esophagus, stomach, and large and small intestine exhibited normal size and location, and pillories and duodenum were open.

Serum from the treated and untreated mice were also tested for the presence of LPS. LPS was not present in serum of mice given oral insulin composition of the present invention + LPS nor in serum of mice given 1ml PBS + LPS , showing that neither the matrix carrier composition nor the gavage compromised the integrity of the mice's gastrointestinal linings. Serum from mice injected 1mg of LPS in 1ml of PBS served as a positive control , and untreated mice served as negative control.

The foregoing description of the specific embodiments will so fully reveal the general nature of the invention that others can, by applying current knowledge, readily modify and/or adapt for various applications such specific embodiments without undue experimentation and without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. The means, materials, and steps for carrying out various disclosed functions may take a variety of alternative forms without departing from the invention.

**Claims**

**1.** A pharmaceutical composition for oral use comprising an oil having particulate matter suspended therein, wherein the particulate matter comprises:

a. a polysaccharide in intimate non-covalent association with silica nanoparticles having a hydrophobic surface, wherein the size of the silica nanoparticles is between 1-100 nanometers; and
b. an insulin protein non-covalently associated with said silica nanoparticles and the polysaccharide.

2. The pharmaceutical composition of claim 1, wherein the polysaccharide comprises a branched polysaccharide.

3. The pharmaceutical composition of claim 2,
wherein said branched polysaccharide is selected from the group consisting of amylopectin, starch and glycogen; or
wherein said branched polysaccharide is a starch; or
wherein said branched polysaccharide has a melting temperature of not more than 400 °C.

4. The pharmaceutical composition of claim 1,
further comprising an additional biopolymer selected from the group consisting of a polysaccharide and a high molecular weight structural protein, wherein said additional biopolymer is a linear biopolymer, preferably wherein said additional biopolymer is a high molecular weight structural protein selected from the group consisting of elastin, collagen, keratin, and fibrinogen and combinations and derivatives thereof; or
wherein said polysaccharide is a linear polysaccharide, preferably wherein said linear polysaccharide is selected from the group consisting of chitin, cellulose, amylose, beta glucan and combinations and derivatives thereof; or
wherein said composition is anhydrous; or
wherein said size of said silica nanoparticles is between 5-30 nanometers; or
wherein said hydrophobic surface of said silica nanoparticles comprises hydrocarbon moieties; or
wherein said polysaccharide is a dietary fiber; or
wherein said silica nanoparticles have a melting temperature of not less than 600 °C; or
wherein said oil comprises a mixture of oils; or
wherein said oil comprises a mixture of oils selected from natural vegetable oils and synthetic analogues thereof; or
wherein said composition further comprises an antioxidant; or
wherein said oil comprises an oil that has a melting temperature of at least 5-10°C; or
further comprising a wax.

5. The pharmaceutical composition of any one of claims 1-4, wherein the weight of said particulate matter is not more than 25% of the weight of said pharmaceutical composition.

6. The pharmaceutical composition of claim 1 for administering insulin to a subject.

7. The pharmaceutical composition of claim 1 for treating diabetes in a subject.

8. Use of the pharmaceutical composition of claim 1 in the preparation of a medicament for administering insulin to a subject.

9. Use of the pharmaceutical composition of claim 1 in the preparation of a medicament for treating diabetes in a subject.

10. A method of manufacturing a pharmaceutical composition formulated for oral delivery of insulin, said method comprising the steps of:

    a. mixing silica nanoparticles having a hydrophobic surface, wherein the size of said silica nanoparticles is between 1-100 nanometers, with a polysaccharide, whereby said silica nanoparticles form an intimate non-covalent association with said polysaccharide;
    b. mixing an insulin protein with an oil; and
    c. mixing said silica nanoparticles and polysaccharide into the oil,

    wherein said insulin forms an intimate non-covalent association with said silica nanoparticles and said polysaccharide and wherein said silica nanoparticles, said polysaccharide and said insulin protein are dispersed in said oil.

11. The method of claim 10,
wherein the polysaccharide comprises a branched polysaccharide; or
further comprising the step of adding an additional oil component following the addition of the oil, preferably wherein said additional oil component has a higher viscosity than said oil; or
further comprising the step of adding a wax following the addition of said oil.

12. The method of claim 10, further comprising the step of adding an additional biopolymer to the mixture of silica nanoparticles and polysaccharide, wherein said additional biopolymer is a linear biopolymer.

**13.** The method of claim 12,
wherein said additional biopolymer is a linear polysaccharide; or
wherein said additional biopolymer is selected from the group consisting of chitin, cellulose, and beta glucan; or
wherein said additional biopolymer is a dietary fiber.

**14.** A method of manufacturing a pharmaceutical composition formulated for oral delivery of insulin, said method comprising the steps of:

a. mixing silica nanoparticles having a hydrophobic surface, wherein the size of said silica nanoparticles is between 1-100 nanometers, with (a) a polysaccharide, and (b) an insulin protein, whereby said silica nanoparticles form an intimate non-covalent association with said polysaccharide and said insulin protein; and
b. mixing said silica nanoparticles, said polysaccharide, and said insulin protein into an oil,

wherein said silica nanoparticles, said polysaccharide, and said insulin protein are suspended in said oil.

**15.** The method of claim 14,
wherein the polysaccharide comprises a branched polysaccharide; or
further comprising the step of adding an additional oil component following the addition of the oil, preferably wherein said additional oil component has a higher viscosity than said oil; or
further comprising the step of adding a wax following the addition of said oil; or
further comprising the step of adding an additional biopolymer selected from the group consisting of a polysaccharide and a high molecular weight structural protein, wherein said additional biopolymer is a linear biopolymer, preferably wherein said additional biopolymer is a polysaccharide, or wherein said additional biopolymer is selected from the group consisting of chitin, cellulose, and beta glucan, or wherein said additional biopolymer is a dietary fiber; or
wherein said insulin is in a dry lyophilized form.

**Patentansprüche**

**1.** Pharmazeutische Zusammensetzung zur oralen Verwendung, umfassend ein Öl mit darin suspendierten Teilchen, worin die Teilchen umfassen:

a. ein Polysaccharid in direkter, nicht-kovalenter Assoziierung mit Siliziumdioxid-Nanoteilchen mit einer hydrophoben Oberfläche, worin die Größe der Siliziumdioxid-Nanoteilchen zwischen 1-100 Nanometern liegt; und
b. ein Insulinprotein, das mit den Siliziumdioxid-Nanoteilchen und dem Polysaccharid nicht-kovalent assoziiert ist.

**2.** Pharmazeutische Zusammensetzung nach Anspruch 1, worin das Polysaccharid ein verzweigtes Polysaccharid umfasst.

**3.** Pharmazeutische Zusammensetzung nach Anspruch 2,
worin das verzweigte Polysaccharid ausgewählt ist aus der Gruppe bestehend aus Amylopektin, Stärke und Glycogen; oder
worin das verzweigte Polysaccharid eine Stärke ist; oder
worin das verzweigte Polysaccharid eine Schmelztemperatur von nicht mehr als 400 °C aufweist.

**4.** Pharmazeutische Zusammensetzung nach Anspruch 1,
ferner ein zusätzliches Biopolymer umfassend, das ausgewählt ist aus der Gruppe bestehend aus einem Polysaccharid und einem Strukturprotein von hohem Molekulargewicht, worin das zusätzliche Biopolymer ein lineares Biopolymer ist, vorzugsweise worin das zusätzliche Biopolymer ein Strukturprotein von hohem Molekulargewicht ist, das ausgewählt ist aus der Gruppe bestehend aus Elastin, Kollagen, Keratin, und Fibrinogen und Kombinationen und Derivaten davon; oder
worin das Polysaccharid ein lineares Polysaccharid ist, vorzugsweise worin das lineare Polysaccharid ausgewählt ist aus der Gruppe bestehend aus Chitin, Cellulose, Amylose, Betaglucan und Kombinationen und Derivaten davon; oder
worin die Zusammensetzung wasserfrei ist; oder
worin die Größe der Siliziumdioxid-Nanoteilchen zwischen 5-30 Nanometern liegt; oder
worin die hydrophobe Oberfläche der Siliziumdioxid-Nanoteilchen Kohlenwasserstoff Reste umfasst; oder

worin das Polysaccharid ein Ballaststoff ist; oder
worin die Siliziumdioxid-Nanoteilchen eine Schmelztemperatur von nicht weniger als 600 °C aufweisen; oder
worin das Öl ein Gemisch von Ölen umfasst; oder
worin das Öl ein Gemisch von Ölen umfasst, die ausgewählt sind unter natürlichen, pflanzlichen Ölen und synthetischen Analogen davon; oder
worin die Zusammensetzung ferner ein Antioxidationsmittel umfasst; oder
worin das Öl ein Öl umfasst, das eine Schmelztemperatur von mindestens 5-10°C aufweist; oder
ferner ein Wachs umfassend.

**5.** Die pharmazeutische Zusammensetzung nach einem der Ansprüche 1-4, worin das Gewicht der Teilchen nicht mehr als 25% des Gewichts der pharmazeutischen Zusammensetzung liegt.

**6.** Pharmazeutische Zusammensetzung nach Anspruch 1 zur Verabreichung von Insulin an ein Individuum.

**7.** Pharmazeutische Zusammensetzung nach Anspruch 1 zur Behandlung von Diabetes in einem Individuum.

**8.** Verwendung der pharmazeutischen Zusammensetzung nach Anspruch 1 zur Herstellung eines Arzneimittels zur Verabreichung von Insulin an ein Individuum.

**9.** Verwendung der pharmazeutischen Zusammensetzung nach Anspruch 1 zur Herstellung eines Arzneimittels zur Behandlung von Diabetes in einem Individuum.

**10.** Verfahren zur Herstellung einer pharmazeutischen Zusammensetzung, die zur oralen Verabreichung von Insulin formuliert ist, wobei das Verfahren die Schritte umfasst:

a. Mischen von eine hydrophobe Oberfläche aufweisenden Siliziumdioxid-Nanoteilchen, wobei die Größe der Siliziumdioxid-Nanoteilchen zwischen 1-100 Nanometern liegt, mit einem Polysaccharid, wodurch die Siliziumdioxid-Nanoteilchen eine direkte, nicht-kovalente Assoziierung mit dem Polysaccharid bilden;
b. Mischen eines Insulinproteins mit einem Öl; und
c. Mischen der Siliziumdioxid-Nanoteilchen und des Polysaccharids in das Öl, wobei das Insulin eine direkte, nicht-kovalente Assoziierung mit den Siliziumdioxid-Nanoteilchen und dem Polysaccharid bildet und wobei die Siliziumdioxid-Nanoteilchen, das Polysaccharid und das Insulinprotein in dem Öl dispergiert werden.

**11.** Verfahren nach Anspruch 10,
wobei das Polysaccharid ein verzweigtes Polysaccharid umfasst; oder
ferner den Schritt umfassend, Hinzufügen einer zusätzlichen Öl-Komponente nach der Zugabe des Öls, vorzugsweise wobei die zusätzliche Öl-Komponente eine höher Viskosität als das Öl aufweist; oder
ferner den Schritt umfassend, Hinzufügen eines Wachses nach der Zugabe des Öls.

**12.** Verfahren nach Anspruch 10, ferner den Schritt umfassend, Hinzufügen eines zusätzlichen Biopolymers zu dem Gemisch der Siliziumdioxid-Nanoteilchen und des Polysaccharids, wobei das zusätzliche Biopolymer ein lineares Biopolymer ist.

**13.** Verfahren nach Anspruch 12,
wobei das zusätzliche Biopolymer ein lineares Polysaccharid ist; oder
wobei das zusätzliche Biopolymer ausgewählt ist aus der Gruppe bestehend aus Chitin, Cellulose, und Betaglucan; oder
wobei das zusätzliche Biopolymer ein Ballaststoff ist.

**14.** Verfahren zur Herstellung einer pharmazeutischen Zusammensetzung, die für die orale Verabreichung von Insulin formuliert ist, wobei das Verfahren die Schritte umfasst:

a. Mischen von eine hydrophobe Oberfläche aufweisenden Siliziumdioxid-Nanoteilchen, wobei die Größe der Siliziumdioxid-Nanoteilchen zwischen 1-100 Nanometern liegt, mit (a) einem Polysaccharid, und (b) einem Insulinprotein, wobei die Siliziumdioxid-Nanoteilchen eine direkte, nicht-kovalente Assoziierung mit dem Polysaccharid und dem Insulinprotein bilden; und
b. Mischen der Siliziumdioxid-Nanoteilchen, des Polysaccharids, und des Insulinproteins in ein Öl,

wobei die Siliziumdioxid-Nanoteilchen, das Polysaccharid, und das Insulinprotein in dem Öl suspendiert sind.

**15.** Verfahren nach Anspruch 14,
wobei das Polysaccharid ein verzweigtes Polysaccharid umfasst; oder
ferner den Schritt umfassend, Hinzufügen einer zusätzlichen Öl-Komponente nach der Zugabe des Öls, vorzugsweise wobei die zusätzliche Öl-Komponente eine höhere Viskosität als das Öl aufweist; oder
ferner den Schritt umfassend, Hinzufügen eines Wachses nach der Zugabe des Öls; oder
ferner den Schritt umfassend, Hinzufügen eines zusätzlichen Biopolymers ausgewählt aus der Gruppe bestehend aus einem Polysaccharid und einem Strukturprotein von hohem Molekulargewicht, wobei das zusätzliche Biopolymer ein lineares Biopolymer ist, vorzugsweise wobei das zusätzliche Biopolymer ein Polysaccharid ist, oder wobei das zusätzliche Biopolymer ausgewählt ist aus der Gruppe bestehend aus Chitin, Cellulose, und Betaglucan, oder wobei das zusätzliche Biopolymer ein Ballaststoff ist; oder
wobei das Insulin in einer trockenen, lyophilisierten Form vorliegt.

## Revendications

**1.** Composition pharmaceutique à usage oral comprenant une huile contenant de la matière particulaire, dans laquelle la matière particulaire comprend :

a. un polysaccharide en association intime non covalente avec des nanoparticules de silice ayant une surface hydrophobe, dans lequel la taille des particules de silice est comprise entre 1 et 100 nanomètres ; et
b. une protéine insuline en association non covalente avec lesdites nanoparticules de silice et le polysaccharide.

**2.** Composition pharmaceutique selon la revendication 1, dans laquelle le polysaccharide comprend un polysaccharide ramifié.

**3.** Composition pharmaceutique selon la revendication 2,
dans laquelle ledit polysaccharide ramifié est choisi dans le groupe constitué par l'amylopectine, l'amidon et le glycogène ; ou
dans laquelle ledit polysaccharide ramifié est un amidon ; ou
dans laquelle ledit polysaccharide ramifié a une température de fusion pas supérieure à 400 °C.

**4.** Composition pharmaceutique selon la revendication 1,
comprenant en outre un biopolymère supplémentaire choisi dans le groupe constitué par un polysaccharide et une protéine structurale de masse moléculaire élevée, dans laquelle ledit biopolymère supplémentaire est un biopolymère linéaire, de préférence dans laquelle ledit biopolymère supplémentaire est une protéine structurale de masse moléculaire élevée choisie dans le groupe constitué par l'élastine, le collagène, la kératine et le fibrinogène ainsi que les combinaisons et les dérivés de ceux-ci ; ou
dans laquelle ledit polysaccharide est un polysaccharide linéaire, de préférence dans laquelle ledit polysaccharide linéaire est choisi dans le groupe constitué par la chitine, la cellulose, l'amylose, le bêta-glucane ainsi que les combinaisons et les dérivés de ceux-ci ; ou
dans laquelle ladite composition est anhydre ; ou
dans laquelle ladite taille desdites nanoparticules de silice est comprise entre 5 et 30 nanomètres ; ou
dans laquelle ladite surface hydrophobe desdites nanoparticules de silice comprend des fragments hydrocarbonés ; ou
dans laquelle ledit polysaccharide est une fibre alimentaire ; ou
dans laquelle lesdites nanoparticules de silice ont une température de fusion pas inférieure à 600 °C ; ou
dans laquelle ladite huile comprend un mélange d'huiles ; ou
dans laquelle ladite huile comprend un mélange d'huiles choisies parmi les huiles végétales naturelles et les analogues synthétiques de celles-ci ; ou
dans laquelle ladite composition comprend en outre un antioxydant ; ou
dans laquelle ladite huile comprend une huile ayant une température de fusion d'au moins 5 à 10 °C ; ou
comprenant en outre une cire.

**5.** Composition pharmaceutique selon l'une quelconque des revendications 1 à 4, dans laquelle la masse de ladite matière particulaire n'est pas supérieure à 25 % de la masse de ladite composition pharmaceutique.

6. Composition pharmaceutique selon la revendication 1 pour administrer de l'insuline à un patient.

7. Composition pharmaceutique selon la revendication 1 pour traiter le diabète chez un patient.

8. Utilisation de la composition pharmaceutique selon la revendication 1 dans la préparation d'un médicament permettant d'administrer de l'insuline à un patient.

9. Utilisation de la composition pharmaceutique selon la revendication 1 dans la préparation d'un médicament permettant de traiter le diabète chez un patient.

10. Procédé de fabrication d'une composition pharmaceutique formulée pour une administration orale d'insuline, ledit procédé comprenant les étapes consistant à :

a. mélanger des nanoparticules de silice ayant une surface hydrophobe, dans lequel la taille desdites nanoparticules de silice est comprise entre 1 et 100 nanomètres, avec un polysaccharide, moyennant quoi lesdites nanoparticules de silice forment une association intime non covalente avec ledit polysaccharide ;
b. mélanger une protéine insuline avec une huile ; et
c. mélanger lesdites nanoparticules de silice et le polysaccharide dans l'huile,

dans lequel ladite insuline forme une association intime non covalente avec lesdites nanoparticules de silice et ledit polysaccharide et dans lequel lesdites nanoparticules de silice, ledit polysaccharide et ladite protéine insuline sont dispersés dans ladite huile.

11. Procédé selon la revendication 10,
dans lequel le polysaccharide comprend un polysaccharide ramifié ; ou
comprenant en outre l'étape consistant à ajouter un composant huileux supplémentaire après l'ajout de l'huile, de préférence dans lequel ledit composant huileux supplémentaire a une viscosité supérieure à celle de ladite huile ; ou
comprenant en outre l'étape consistant à ajouter une cire après l'ajout de ladite huile.

12. Procédé selon la revendication 10, comprenant en outre l'étape consistant à ajouter un biopolymère supplémentaire dans le mélange de nanoparticules de silice et de polysaccharide, dans lequel ledit biopolymère supplémentaire est un biopolymère linéaire.

13. Procédé selon la revendication 12,
dans lequel ledit biopolymère supplémentaire est un polysaccharide linéaire ; ou
dans lequel ledit biopolymère supplémentaire est choisi dans le groupe constitué par la chitine, la cellulose et le bêta-glucane ; ou
dans lequel ledit biopolymère supplémentaire est une fibre alimentaire.

14. Procédé de fabrication d'une composition pharmaceutique formulée pour une administration orale d'insuline, ledit procédé comprenant les étapes consistant à :

a. mélanger des nanoparticules de silice ayant une surface hydrophobe, dans lequel la taille desdites nanoparticules de silice est comprise entre 1 et 100 nanomètres, avec (a) un polysaccharide et (b) une protéine insuline, moyennant quoi lesdites nanoparticules de silice forment une association intime non covalente avec ledit polysaccharide et ladite protéine insuline ; et
b. mélanger lesdites nanoparticules de silice, ledit polysaccharide et ladite protéine insuline dans une huile,

dans lequel lesdites nanoparticules de silice, ledit polysaccharide et ladite protéine insuline sont en suspension dans ladite huile.

15. Procédé selon la revendication 14,
dans lequel le polysaccharide comprend un polysaccharide ramifié ; ou
comprenant en outre l'étape consistant à ajouter un composant huileux supplémentaire après l'ajout de l'huile, de préférence dans lequel ledit composant huileux supplémentaire a une viscosité supérieure à celle de ladite huile ; ou
comprenant en outre l'étape consistant à ajouter une cire après l'ajout de ladite huile ; ou
comprenant en outre l'étape consistant à ajouter un biopolymère supplémentaire choisi dans le groupe constitué par un polysaccharide et une protéine structurale de masse moléculaire élevée, dans lequel ledit biopolymère

supplémentaire est un biopolymère linéaire, de préférence dans lequel ledit biopolymère supplémentaire est un polysaccharide, ou dans lequel ledit biopolymère supplémentaire est choisi dans le groupe constitué par la chitine, la cellulose et le bêta-glucane, ou dans lequel ledit biopolymère supplémentaire est une fibre alimentaire ; ou dans lequel ladite insuline est sous forme lyophilisée sèche.

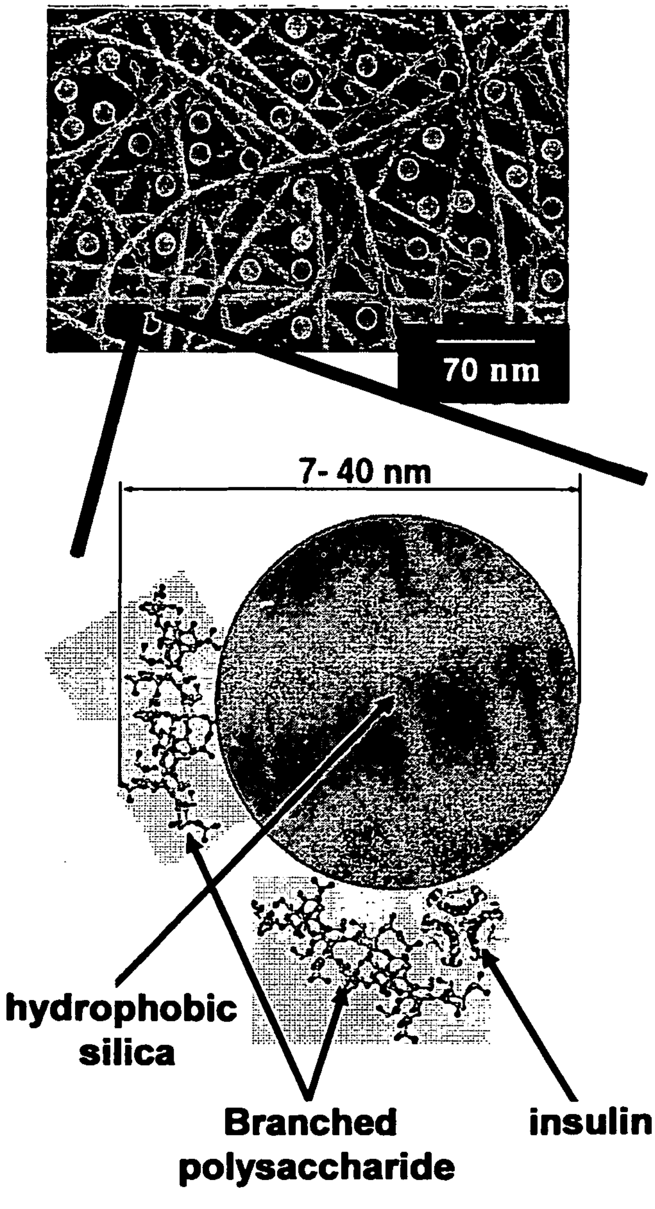
70 nm
7- 40 nm
hydrophobic
silica
Branched
polysaccharide
insulin

Figure 1

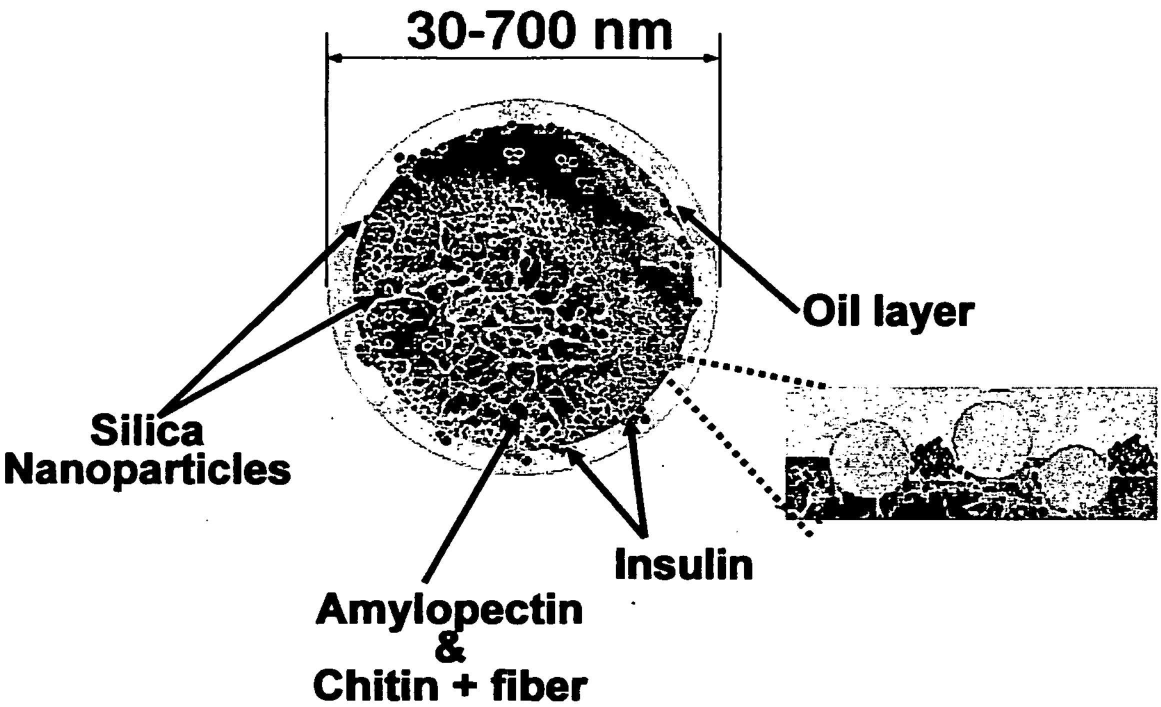
30-700 nm
Oil layer
Silica
Nanoparticles
Insulin
Amylopectin
&
Chitin + fiber

Figure 2

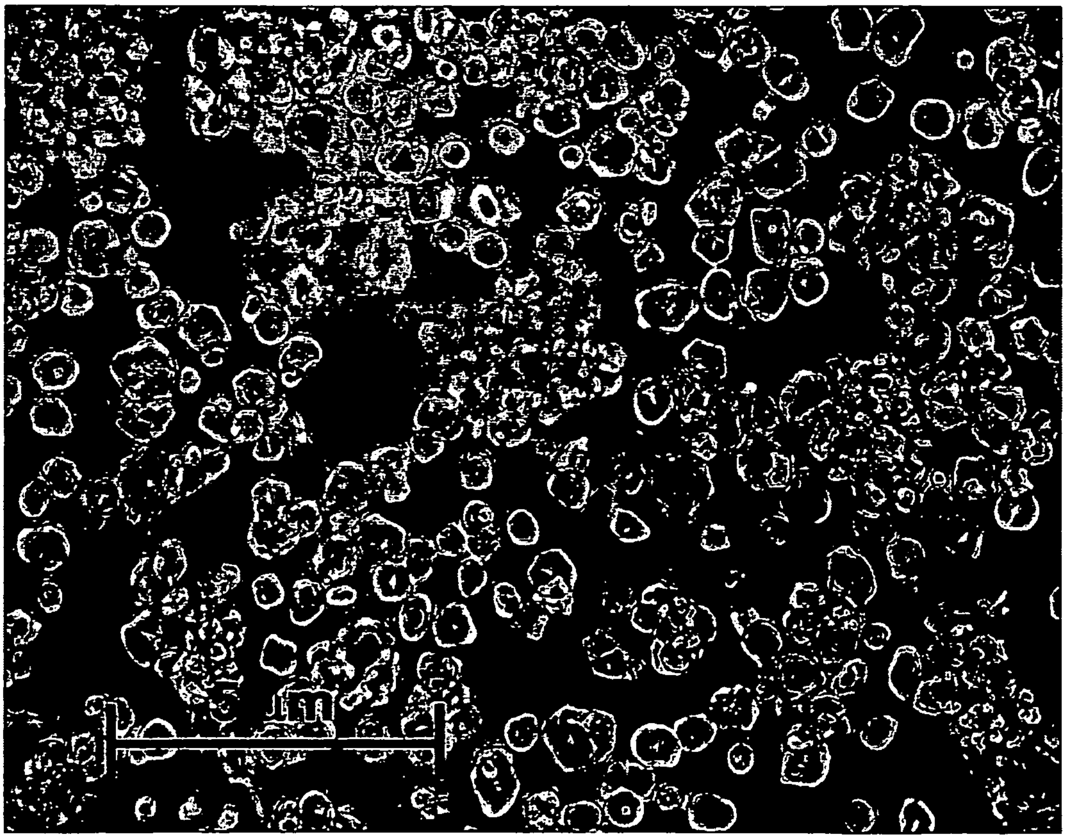

Figure 3

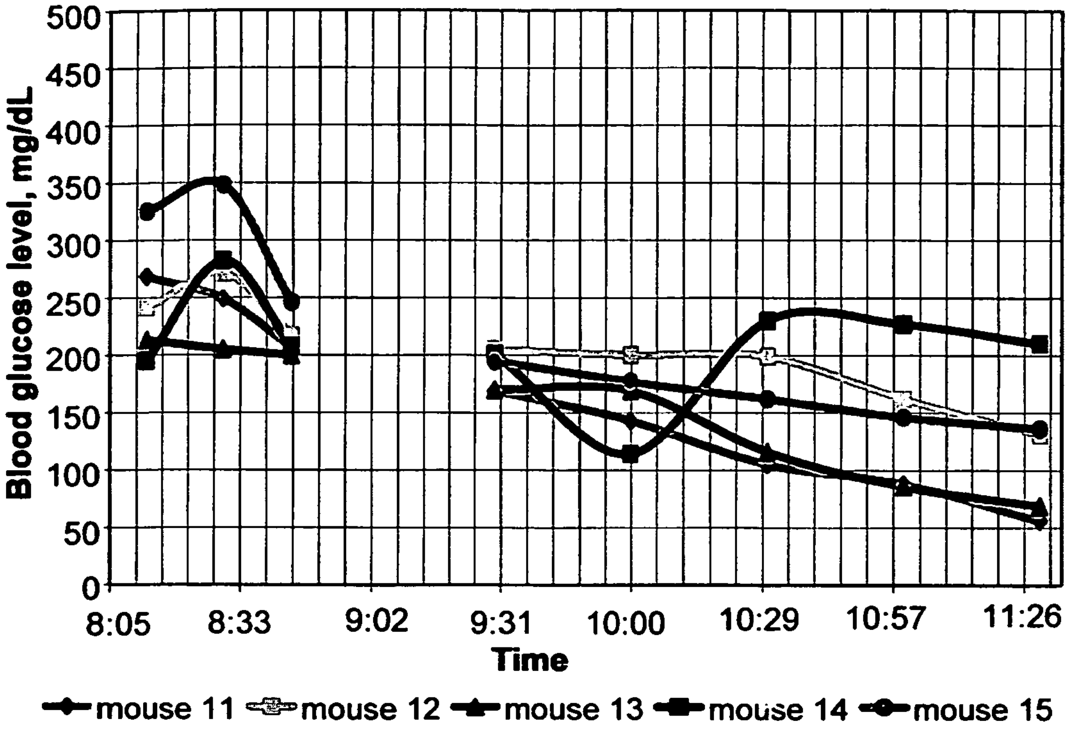


Figure 4A

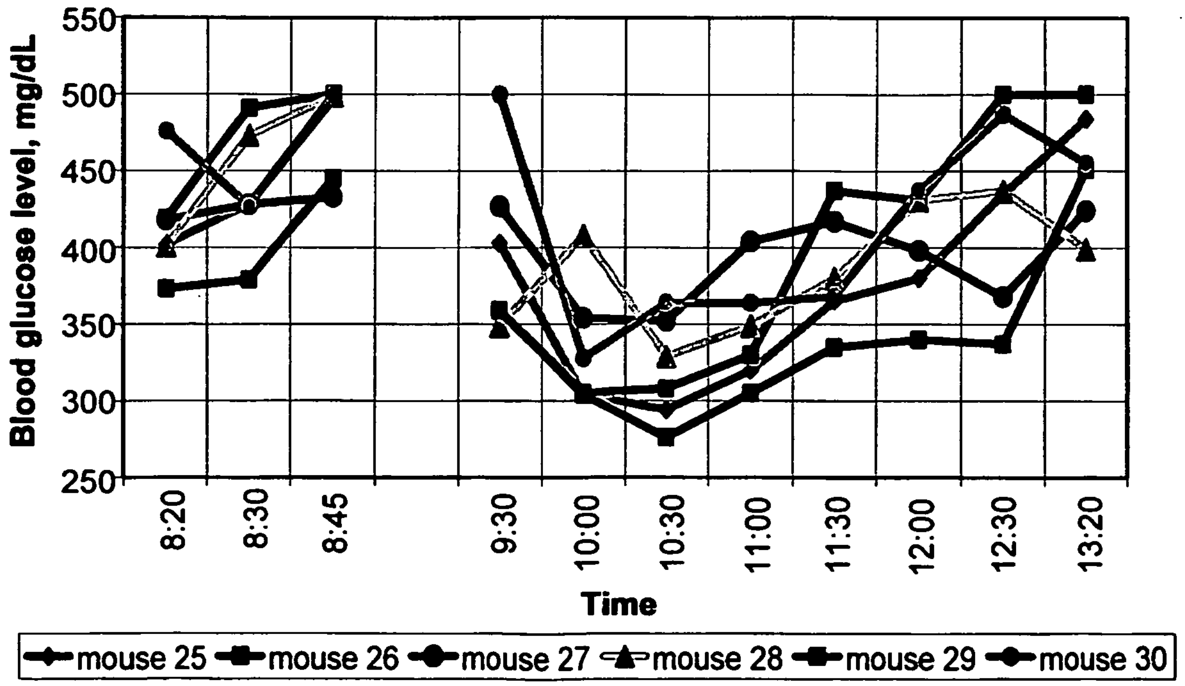


Figure 4B

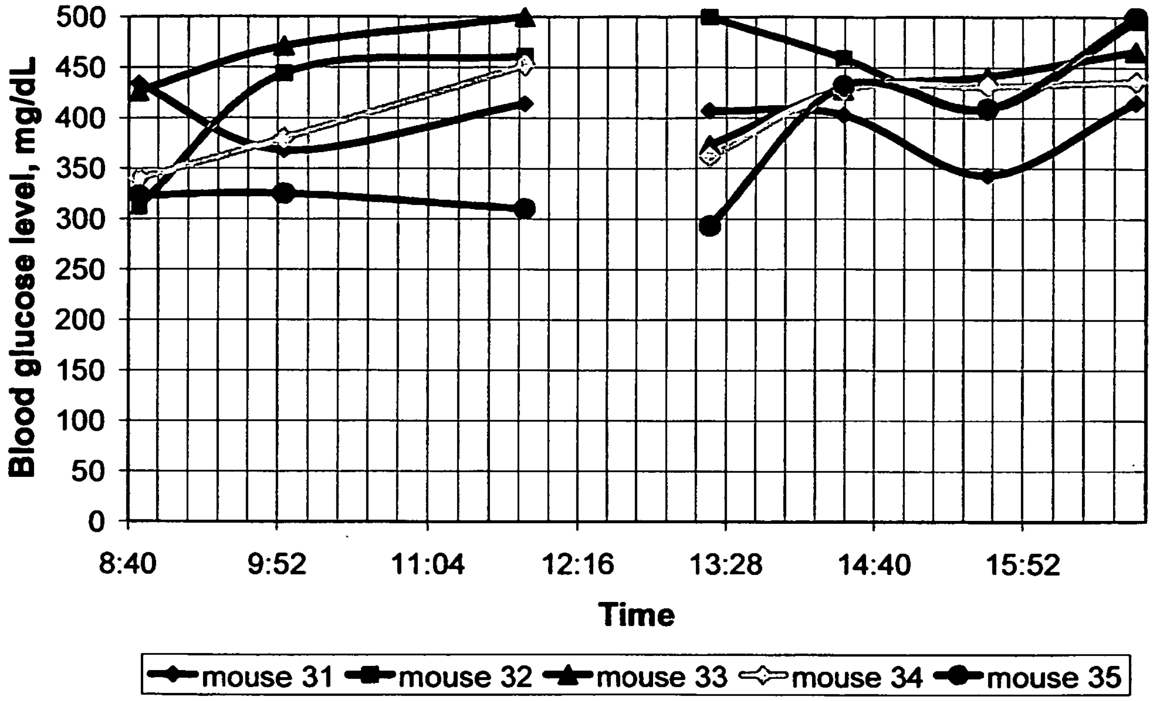
Blood glucose level, mg/dL
500
450
400
350
300
250
200
150
100
50
0
8:40
9:52
11:04
12:16
13:28
14:40
15:52
Time
mouse 31
mouse 32
mouse 33
mouse 34
mouse 35

Figure 5

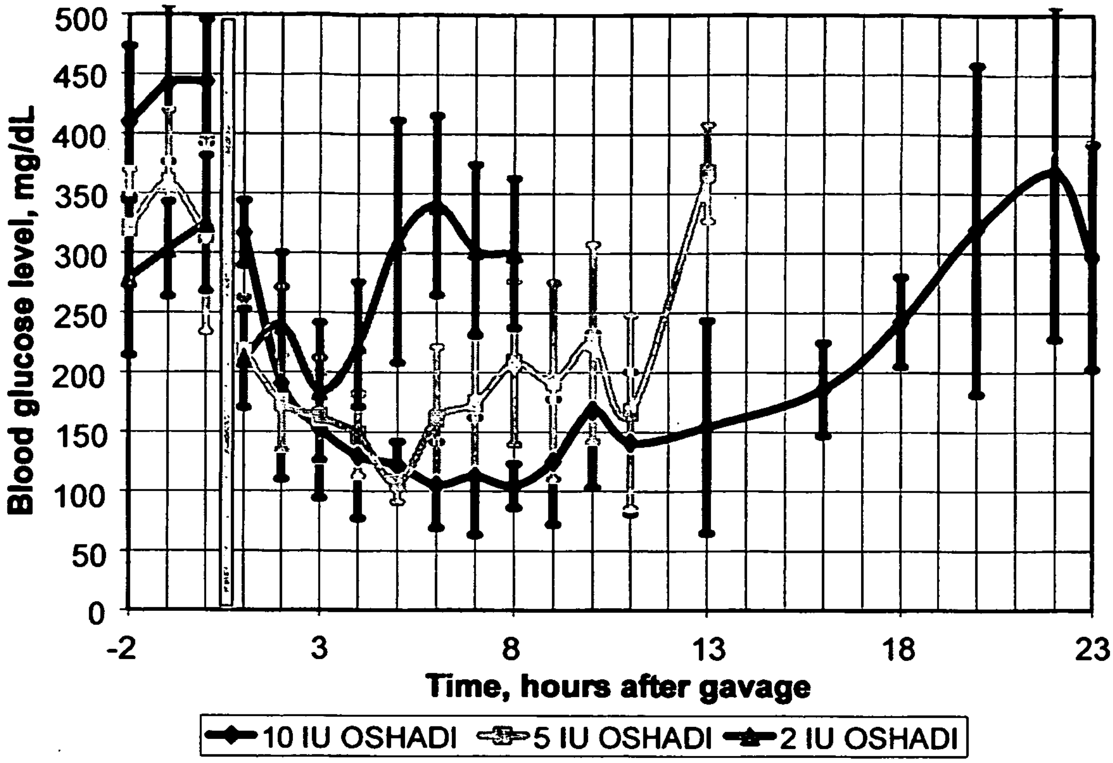


Figure 6A

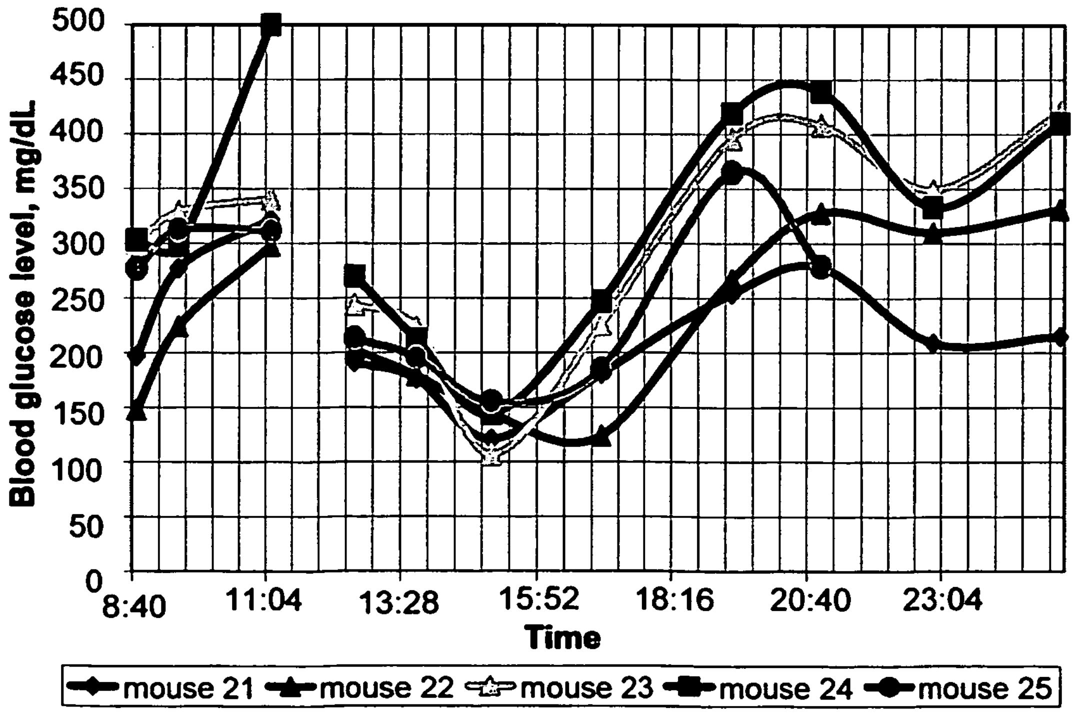


Figure 6B

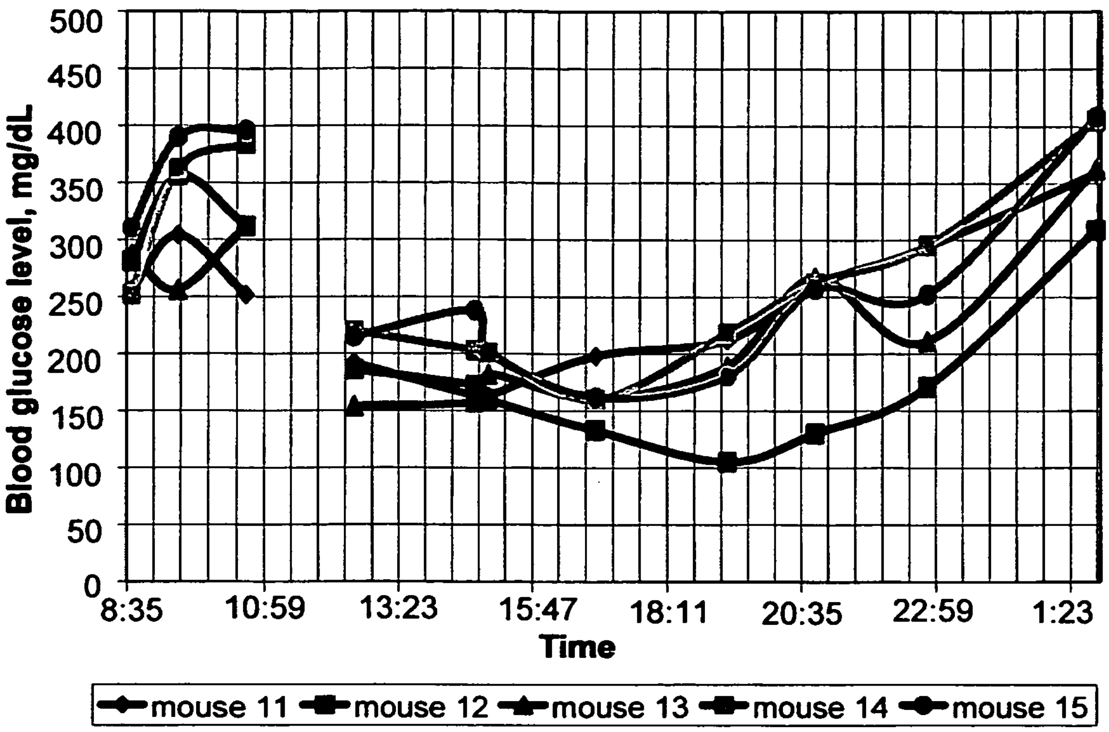


Figure 6C

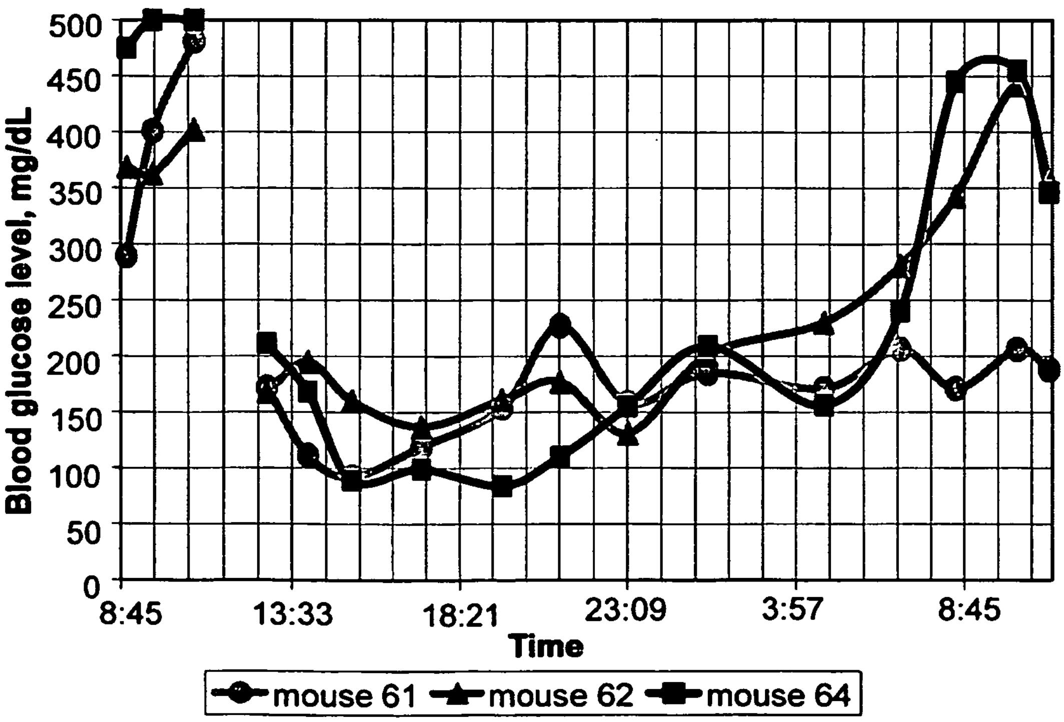


Figure 6D

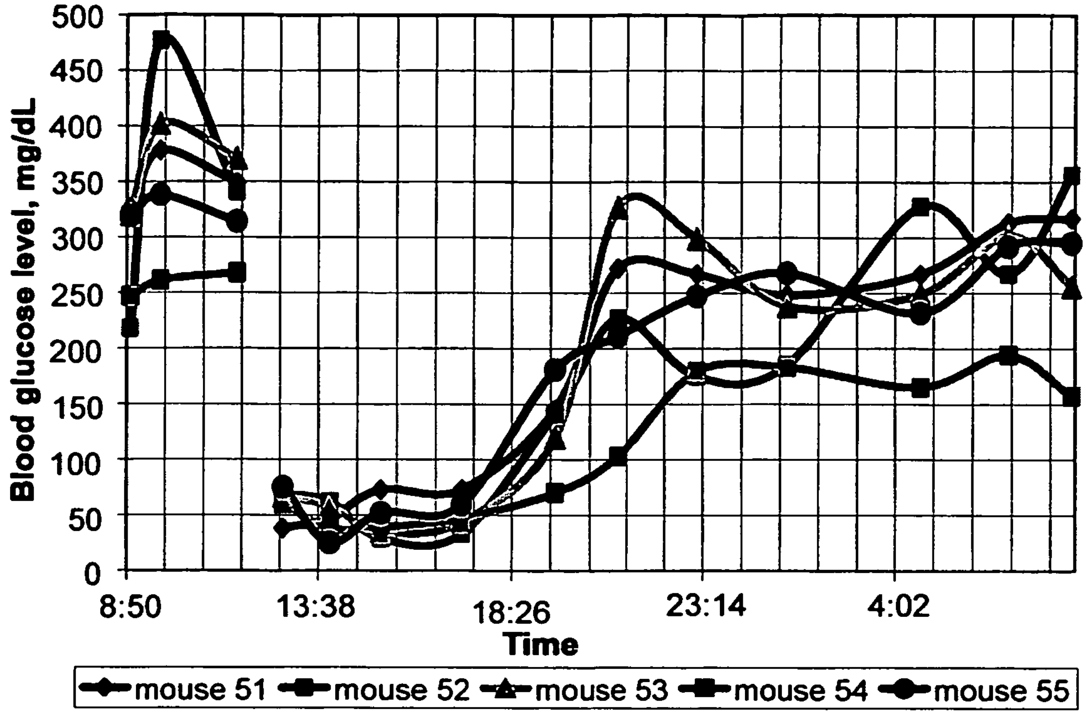


Figure 6E

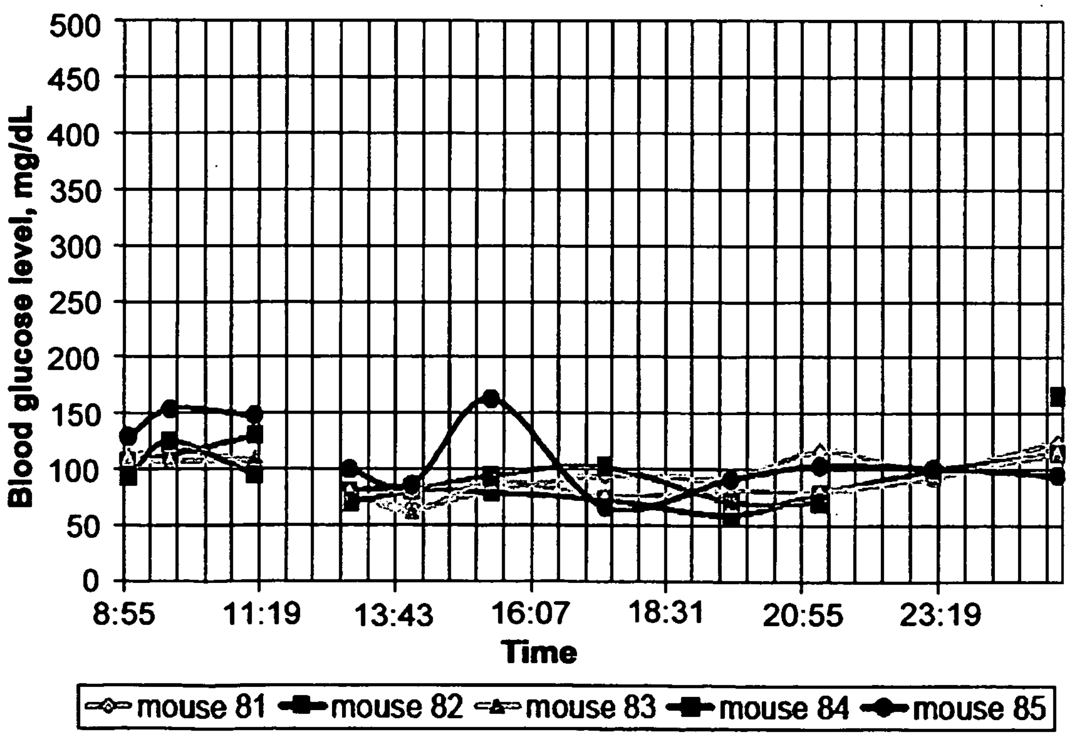


Figure 6F

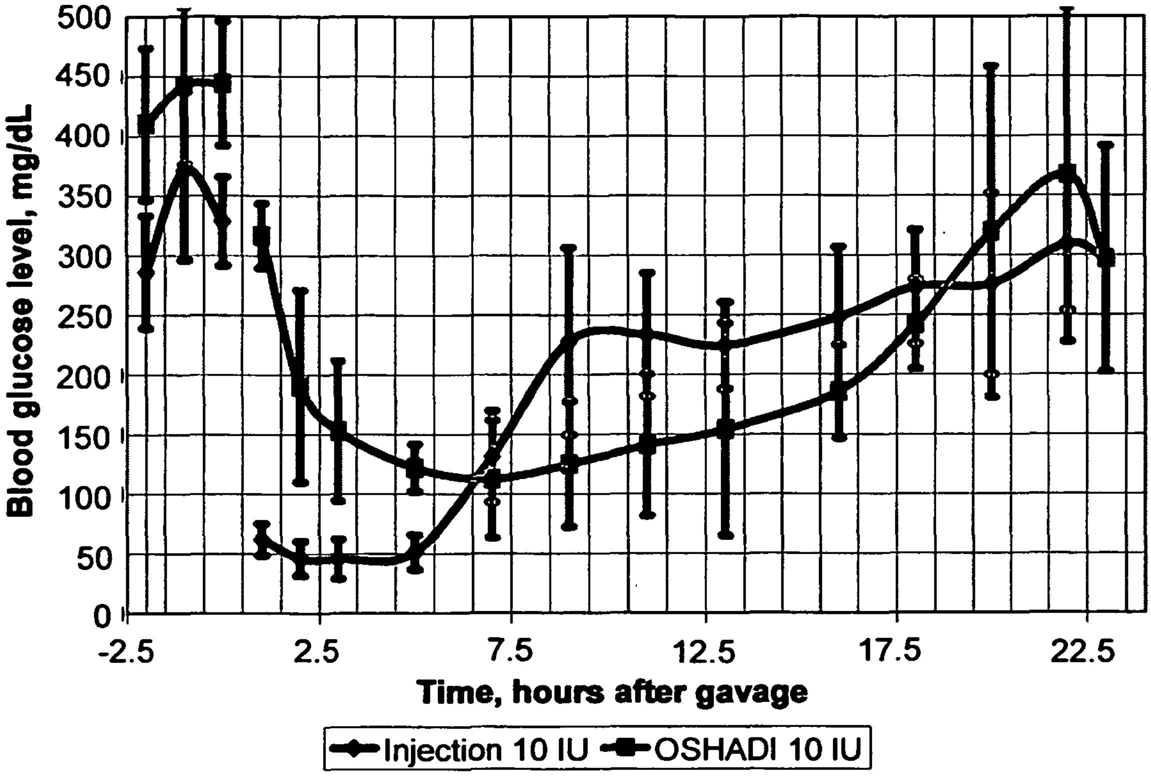
500
450
400
350
300
250
200
150
100
50
0
Blood glucose level, mg/dL
-2.5
2.5
7.5
12.5
17.5
22.5
Time, hours after gavage
Injection 10 IU
OSHADI 10 IU

Figure 6G

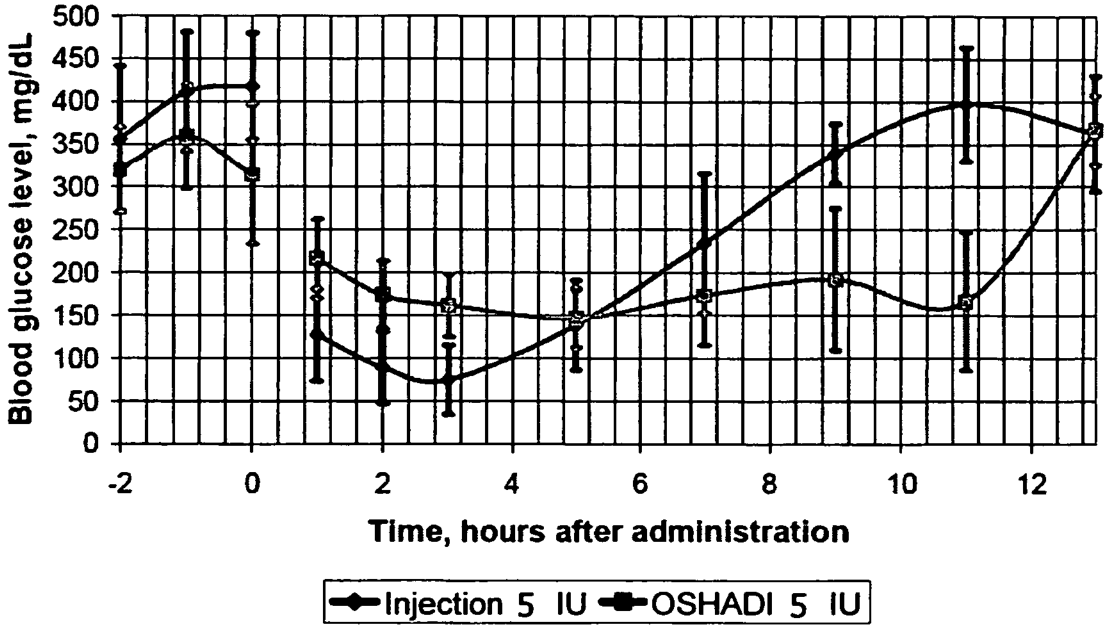


Figure 6H

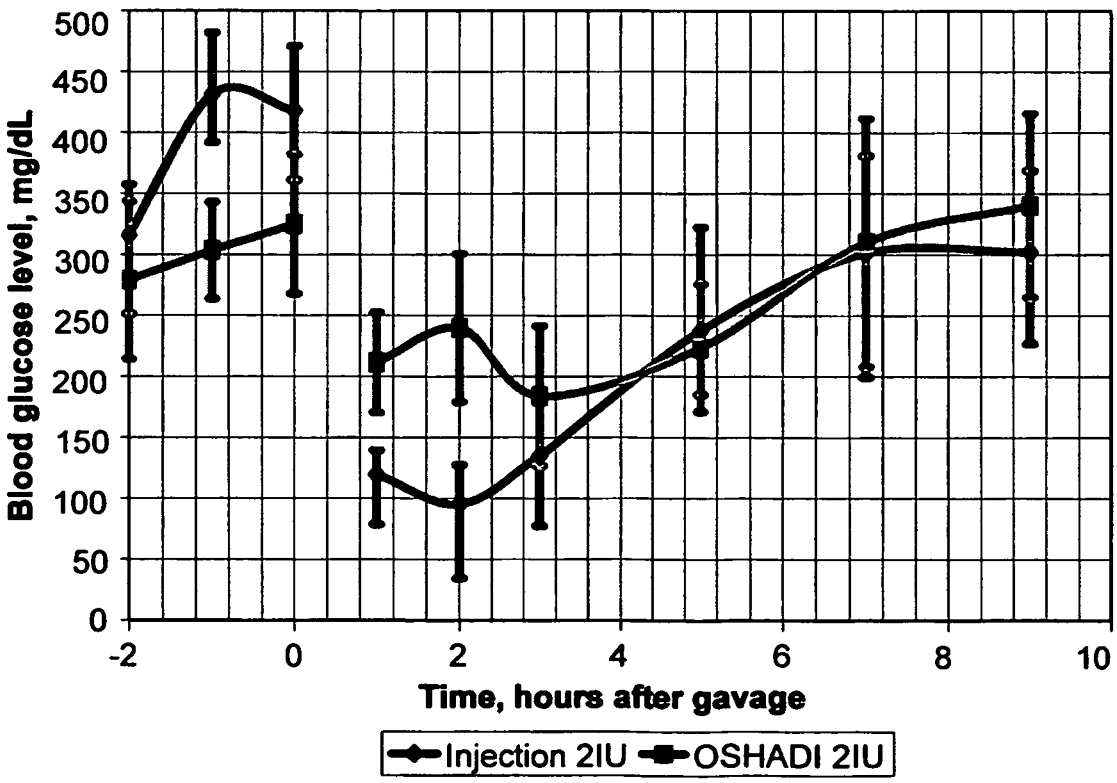


Figure 6I

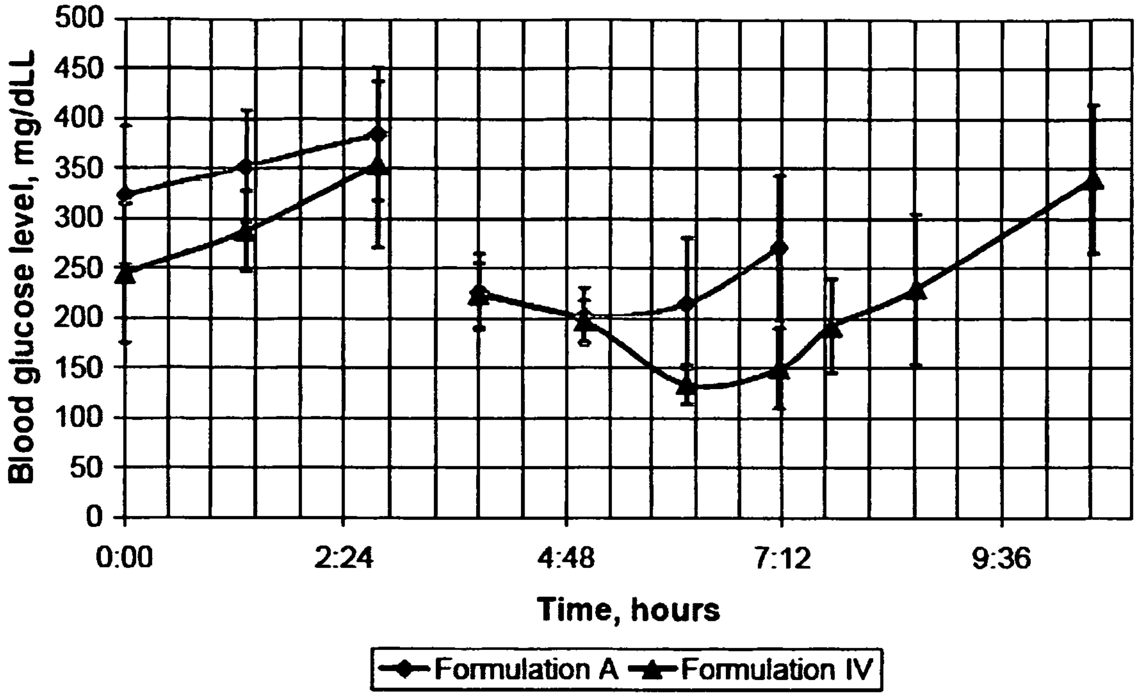


Figure 6J

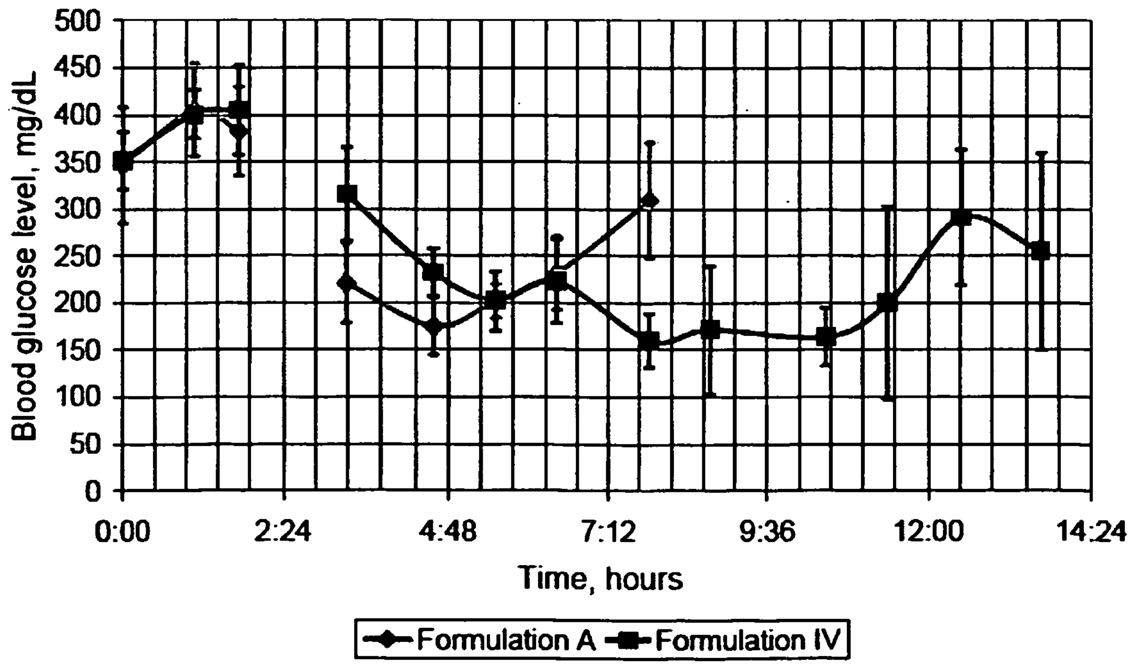


Figure 6K

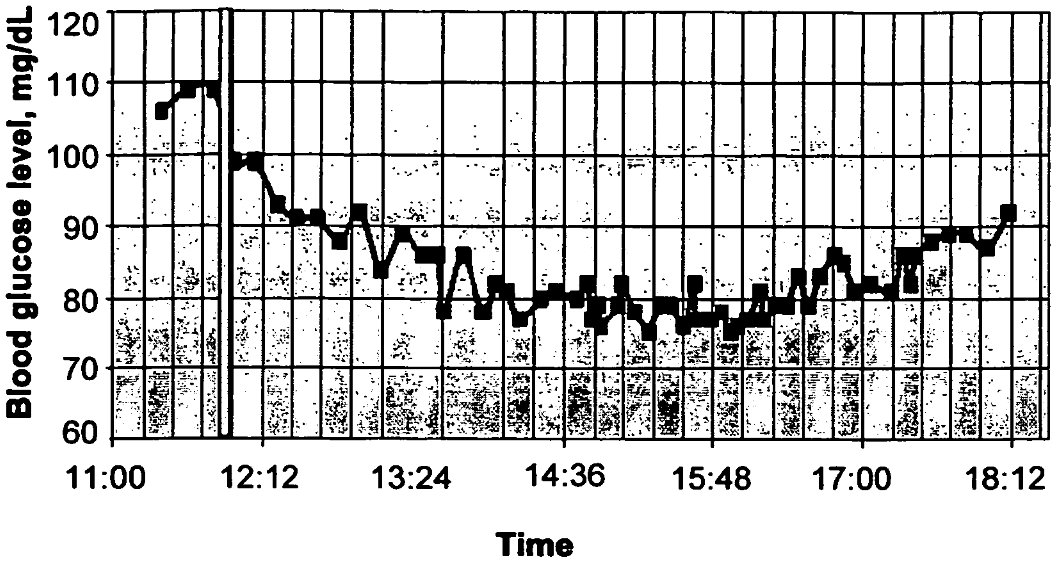


Figure 7A

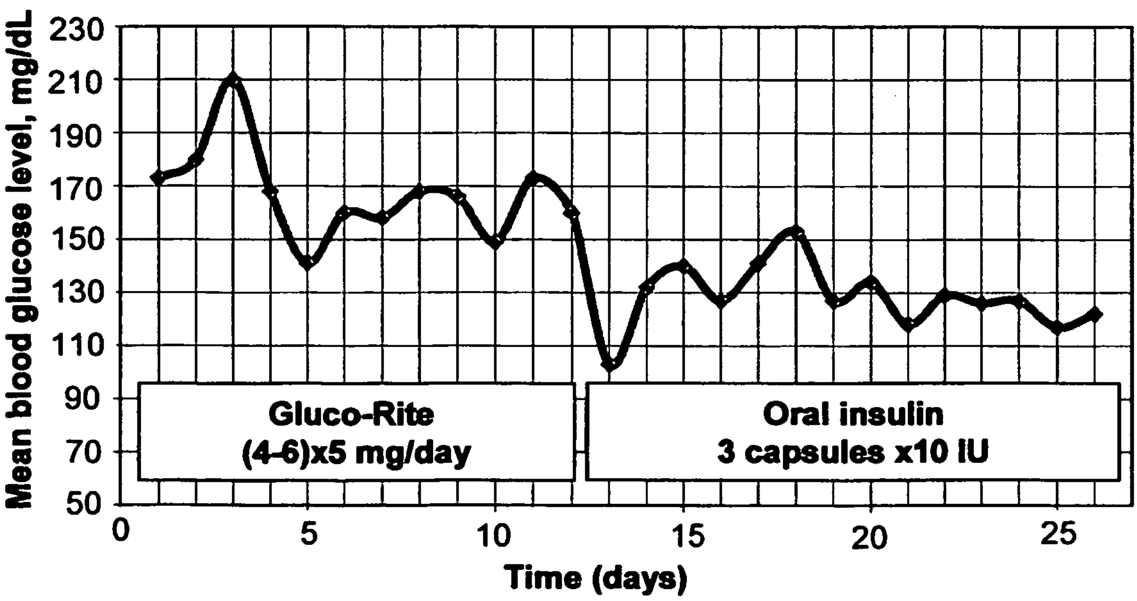


Figure 7B

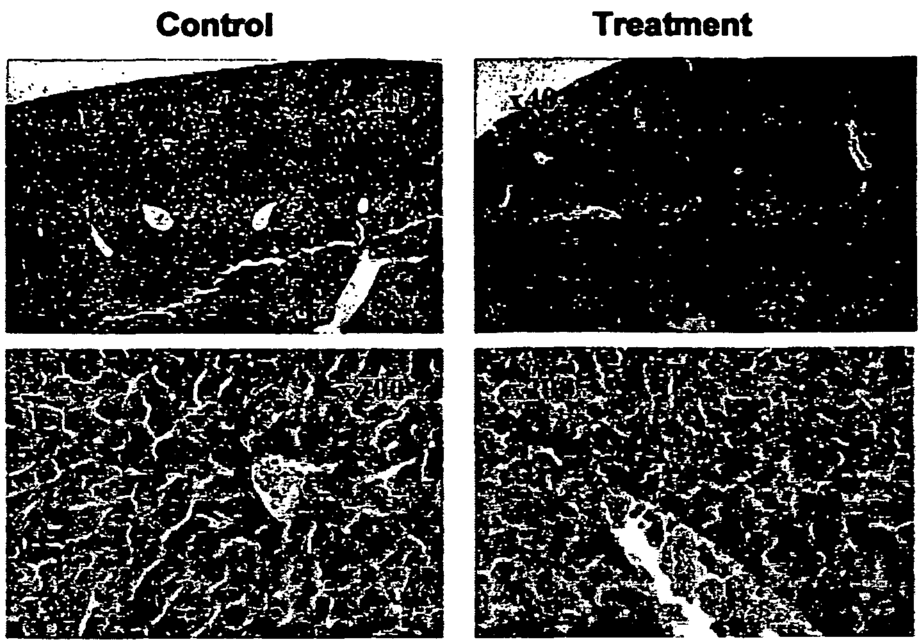


Figure 8A

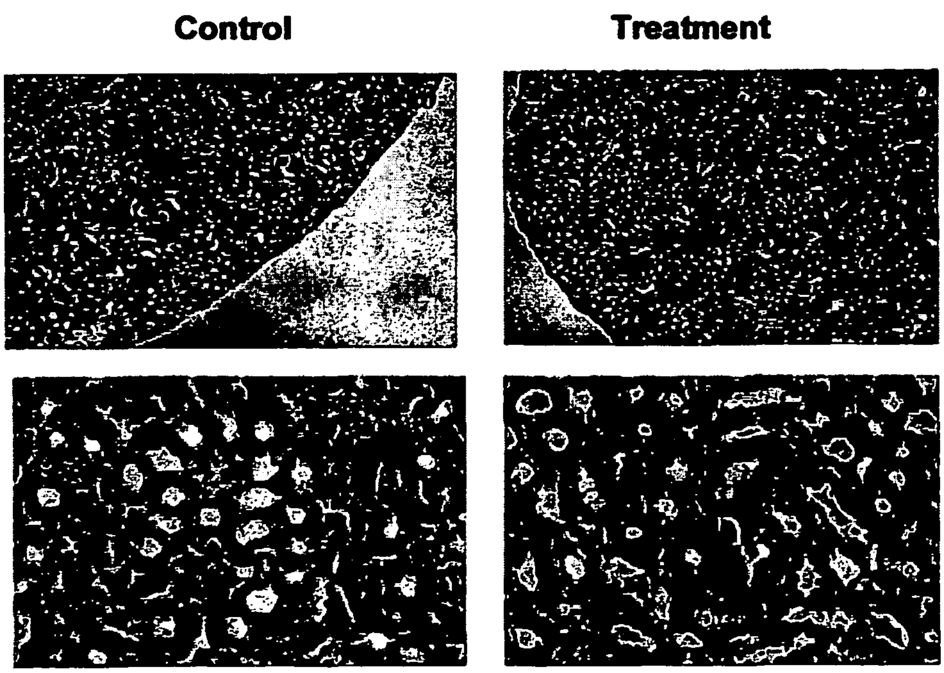


Figure 8B

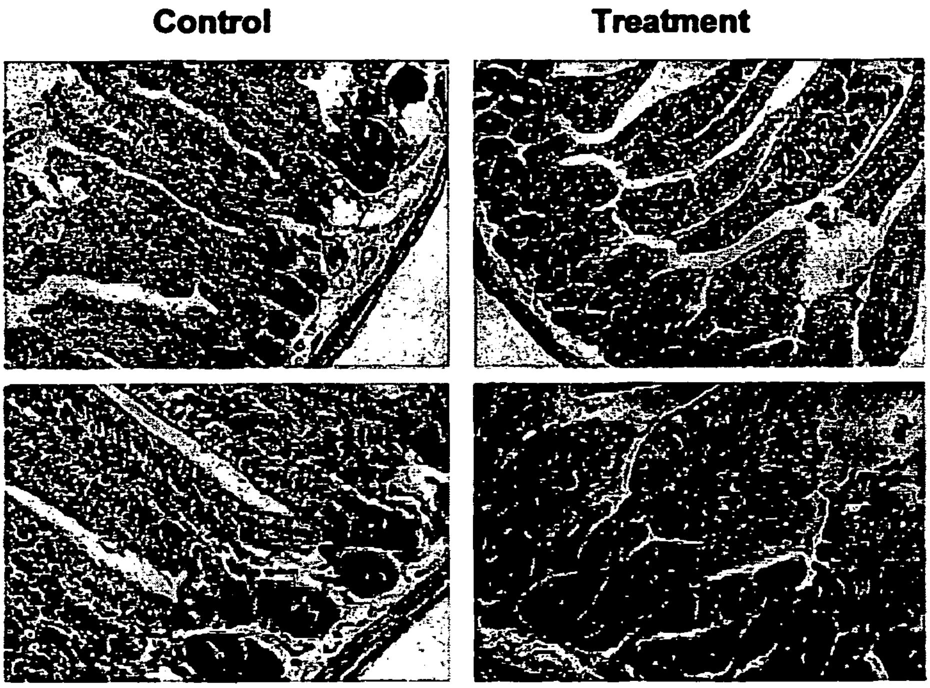


Figure 8C

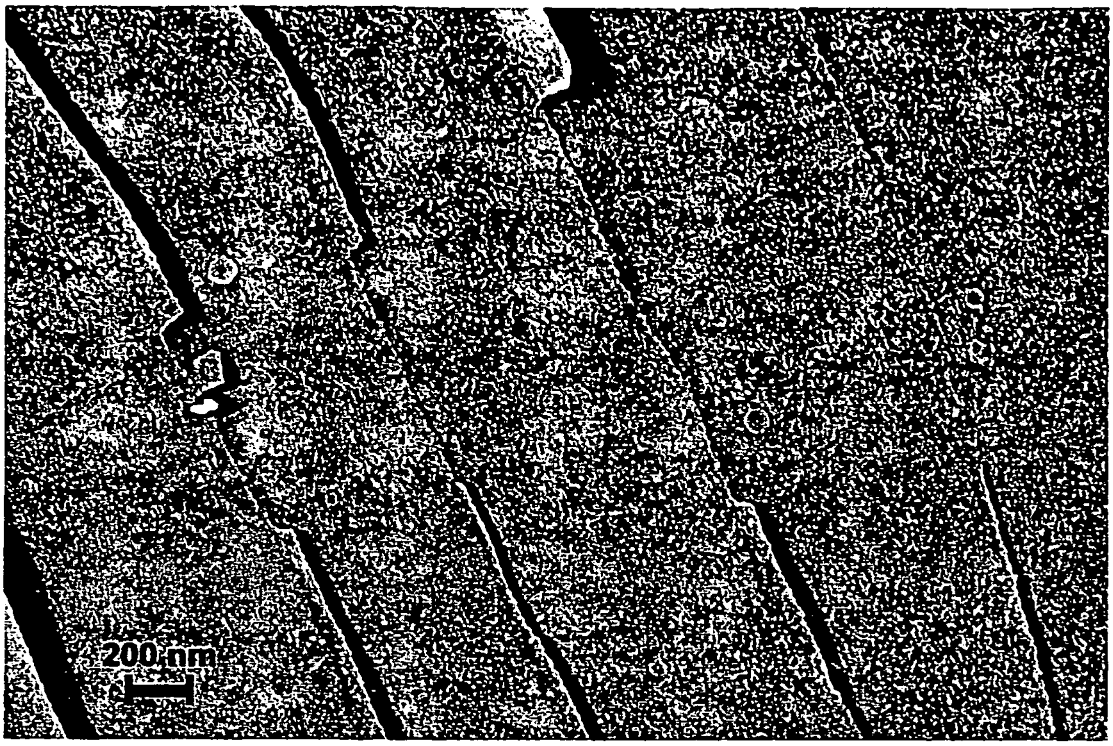


Figure 9A

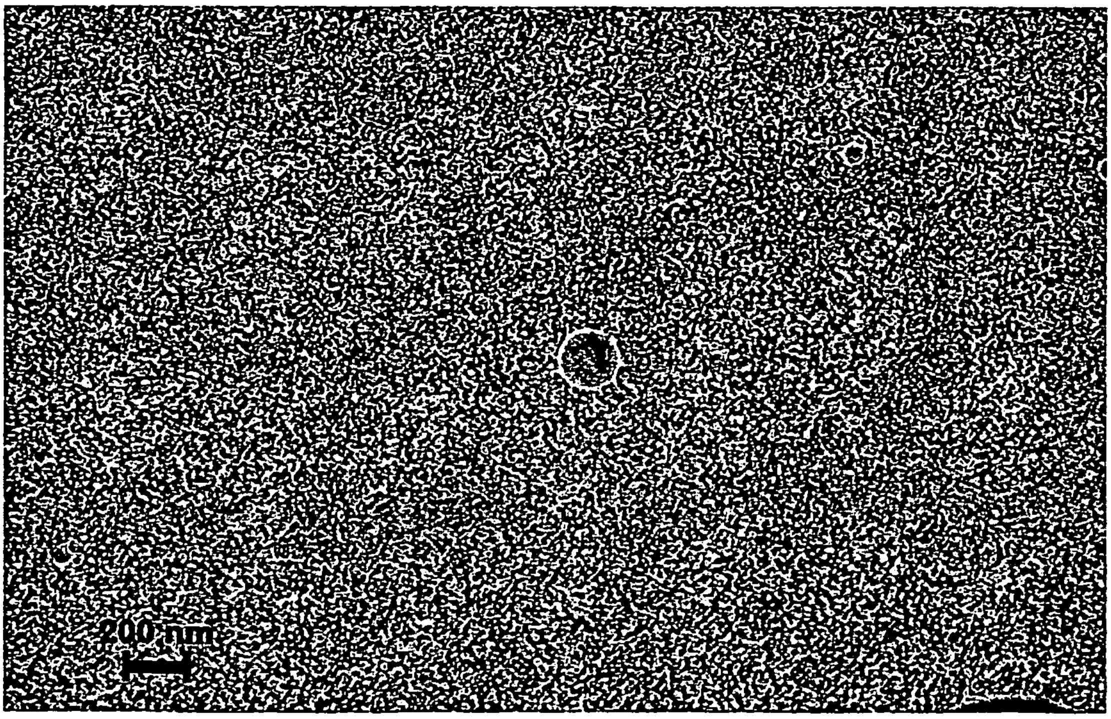


Figure 9B

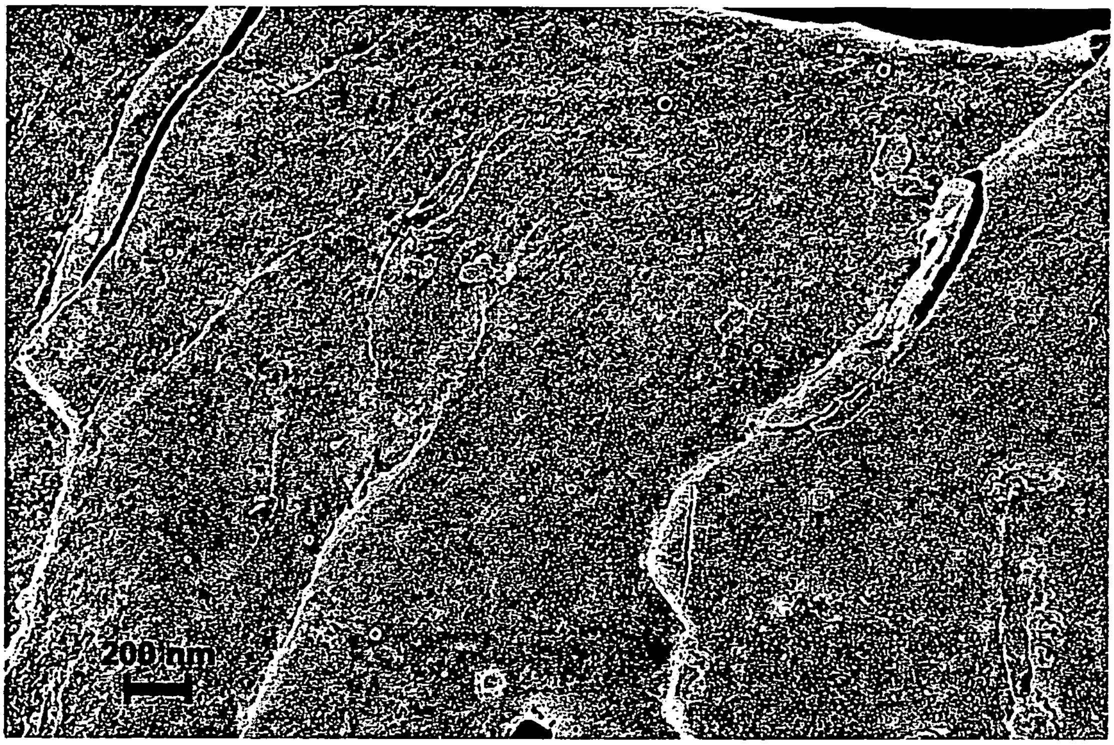


Figure 9C

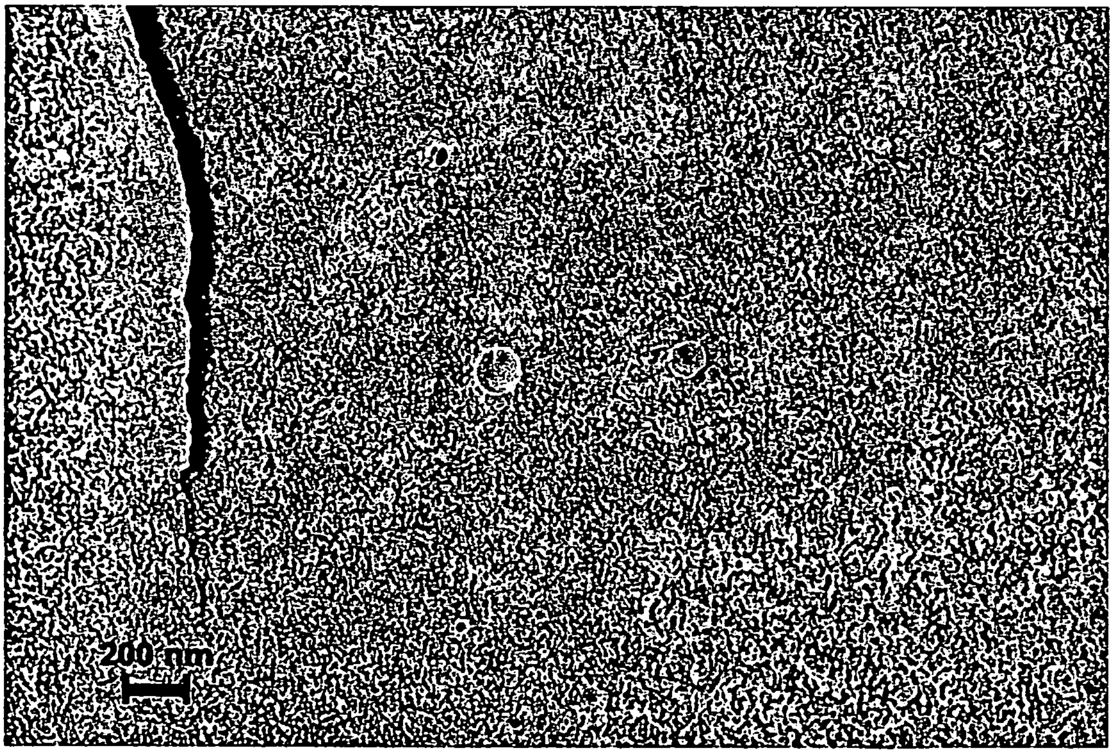


Figure 9D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7455830 B
- US 7470663 B
- US 7384914 B
- US 6656922 B
- US 7351741 B, Weidner
- US 6667060 B, Vandecruys
- US 20040115264 A, Blouquin
- US 6322765 B, Muhlhofer
- US 6698247 B, Tennent
- US 7105229 B
- US 6989195 B
- US 6482517 B
- US 6638621 B
- US 6458387 B
- US 7045146 B
- US 5462866 A
- US 20070154559 A, Pai
- US 20060177495 A
- US 20030235619 A, Allen
- US 20060083781 A, Shastri
- WO 9637232 A, Alonso Fernandez
- US 6548264 B, Tan
- US 20070275969 A, Gurny
- US 6228377 B
- US 7090868 B, Gower
- US 7195780 B, Dennis
- US 7316818 B
- WO 06062544 A
- US 6071535 A
- US 5874105 A, Watkins
- US 6551576 B
- US 6808720 B
- US 7083572 B, Unger
- US 20070184076 A
- WO 06097793 A
- WO 05094785 A
- WO 03066859 A, Ben-Sason
- EP 0491114 B1, Guerrero Gomez-Pamo
- US 20070172426 A
- US 20060053971 A
- US 20070098990 A
- US 6528497 B


### Non-patent literature cited in the description


- **CHUNG et al.** Hydrophobic modification of silica nanoparticle by using aerosol spray reactor. *Colloids and Surfaces A: Physicochem. Eng. Aspects,* 2004, vol. 236, 73-79
- **FU X ; QUTUBUDDIN S.** *Colloids Surf. A: Physicochem. Eng. Aspects,* 2001, vol. 179, 65
- **KRYSZTATKIEWICZ A ; JESIONOWSKI T ; BINKOWSKI S.** *Colloids Surf. A: Physicochem. Eng. Aspects,* 2000, vol. 173, 73
- **JEAN J ; YANG S.** *J. Am. Ceram. Soc.,* 2000, vol. 83 (8), 1928
- **ZHANG J ; GAO L.** *Ceram. Int.,* 2001, vol. 27, 143
- Thirteenth report of the Joint FAO/WHO Expert Committee on Food Additives, FAO Nutrition Meetings Report Series. *FAO/WHO Expert Committee on Food Additives meeting in Rome,* 27 May 1969
- CTFA. 1995
- **KRYSZTAFKIEWICZ A ; JESIONOWSKI T ; BINKOWSKI S.** *Colloids Surf. A: Physicochem. Eng. Aspects,* 2000, vol. 173, 73
- **OKADA M. et al.** *Polymer journal,* 1983, vol. 15 (11), 821-26
- **ELEFTHERIADOU I. et al.** The effects of medications used for the management of diabetes and obesity on postprandial lipid metabolism. *Curr Diabetes Rev,* 2008, vol. 4 (4), 340-56
- **VAIDYA HB et al.** Glucagon like peptides-1 modulators as newer target for diabetes. *Curr. Drug Targets,* 2008, vol. 9 (10), 911-20
- **VERVERIDIS F. et al.** Biotechnology of flavonoids and other phenylpropanoid-derived natural products. Part I: Chemical diversity, impacts on plant biology and human health and Part II. *Reconstruction of multienzyme pathways in plants and microbes,* 2007, vol. 2 (10), 1214-49
- Handbook of Pharmaceutical Excipients. Pharmaceutical Press, 2005